# EUROPEAN PATENT APPLICATION

(11) **EP 1 630 226 A2**
(43) Date of publication of application: **01.03.2006**
(21) Application number: 05018383.9
(22) Date of filing: 24.08.2005
(51) Int. Cl.: C12N 9/02, C12N 9/06, C12N 1/21, A23K 1/18, A23K 1/00

(54) **Methane production inhibitor and feed composition for ruminants**

(30) Priority: 25.08.2004 JP 2004245705
(71) Applicant: Ajinomoto Co., Inc., Tokyo 104-8315 (JP)
(72) Inventor: Toride, Yasuhiko, Kawasaki-shi, Kanagawa 210-8681 (JP); Takahashi, Junichi, Obihiro-shi, Hokkaido 080-8555 (JP); Isogai, Nahoko, Kawasaki-shi, Kanagawa 210-8681 (JP); Kuwabara, Yoko, Kawasaki-shi, Kanagawa 210-8681 (JP)
(74) Representative: HOFFMANN EITLE

(57) **Abstract**

This invention provides a feed composition for ruminants comprising a microbe having nitrite reductase active, wherein said microbe is selected from the group consisting of intestinal bacteria, coryneform bacteria, *Bacillus subtilis,* bacteria of the genus *Methylophilus, Actinomyces,* ruminal bacteria, and combination thereof; a method of improving the growth of a ruminant by administering the above feed composition to said ruminant; and a method of preventing nitrite intoxication by administering the above feed composition to a ruminant.

## Description

### BACKGROUND OF THE INVENTION

### Field of the invention

The present invention relates to a methane production inhibitor and a feed composition for ruminants, and more specifically, to a methane production inhibitor for ruminants characterized by comprising a microbe-derived nitrite reductase as an active ingredient, and to a feed composition for ruminants characterized by comprising this methane production inhibitor.

### Background of the related art

The methane produced in the rumen of a ruminant not only constitutes inefficient use of the energy in feed, but is also a greenhouse gas contributing to global warming. Thus, it is highly important to reduce methane production.

Fermentation is conducted in the rumen by a variety of microbes, resulting in production various metabolites. One of these, methane, is thought to be produced by methane producing bacteria. The methane producing bacteria in the rumen are hydrogen-assimilating bacteria that utilize hydrogen to reduce carbon dioxide, generating methane. Accordingly, the presence of a more powerful reduction reaction inhibits methane production (Japanese Patent Application Publication No. 2003-88301, International Patent Application Publication No. WO96/39860).

The administration of ionophores such as monensin and iberin to ruminants is a known method of inhibiting methane production in the rumen. The supplying of cysteine (Japanese Patent Application Publication No. Heisei 7-322828) and the supplying of fumaric acid (Japanese Patent Application Publication No. Heisei 11-46694) are known methods that have received attention in the adjustment of reduction capability in the rumen. It is also known that reduction of nitrates via nitrites and hydroxyamines to ammonia decreases the production of methane in the rumen. However, since nitrite reduction activity is rate-determining in the rumen (Iwamoto M., Asanuma N., Anim Sci J, 1972, 70: 471-478), it is necessary to rapidly reduce nitrites to inhibit methane production. Furthermore, in recent years, the nitrate content of feeds has increased due to the cultivation of forage with nitrogen fertilizer, which creates the problem of large amounts of nitrites accumulate in the rumen.

The toxicity of nitrites accumulating in the rumen is known to decrease the activity of microbes in the rumen (ruminal bacteria) and cause nitrite intoxication in animals. It has become clear that the addition of nitrates to mixed culture systems of ruminal bacteria in an attempt to inhibit the production of methane in the rumen due to nitrate reduction results in an increase in the number of microbes capable of reducing nitrates that are constantly present in the rumen (Iwamoto M., Asanuma N., and Hino T., Anaerobe, 2002, 8, 209-215). Furthermore, methane production inhibitors, including *Propionibacterium acidipropionici,* which has the ability to reduce nitrites, and methane production inhibitors, including microbes derived from sheep's milk such as lactobacilli and yeast, have been developed (U.S. Patent No. 6,120,810, Japanese Patent Application Publication No. 2003-88301).

Microbes such as *Escherichia coli (E. coli)* are known to produce nitrite reductases (nirBD, *nrf*ABCDEFG, Cole, J., FEMS Microbiology Letters Volume 136, February, 1996, Issue 1, Henian Wang and Robert P. Gunsalus, Journal of Bacteriology, 2000, October, p.5813-5822, Vol. 182, No. 20), but a feed composition containing *E. coli* or any other microbe enhancing nitrite acid reducing capability has not been previously developed.

### SUMMARY OF THE INVENTION

Accordingly, the objects of the present invention include providing a methane production inhibitor and a feed composition which inhibits the production of methane in the rumen of a ruminant, enhances feed efficiency, and/or prevents nitrite intoxication. Furthermore, it is an object of the present invention to provide a method of administering the methane production inhibitor or the feed composition to ruminants.

The present inventors conducted extensive research into solving the above-stated problem, resulting in the discovery that the administration of a microbe-derived nitrite reductase into the rumen fluid of a ruminant, or oral administration of the same, enhanced nitrite reduction activity and inhibited methane production in the rumen; the present invention was devised on this basis.

It is an object of the present invention to provide a feed composition for ruminants comprising a microbe having nitrite reductase activity, wherein said microbe has been modified so that intracellular nitrite reductase activity is enhanced.

It is a further object of the present invention to provide the above feed composition, wherein said microbe is selected from the group consisting of intestinal bacteria, coryneform bacteria, *Bacillus bacterium,* bacteria of the genus *Methylophilus, Actinomyces,* ruminal bacteria, and a combination thereof..

It is a further object of the present invention to provide the above feed composition,wherein said microbe is Escherichia bacterium.

It is a further object of the present invention to provide the above feed composition, wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20, 22, and combination thereof, except that 1 to 30 amino acids have been substituted, deleted, inserted, or added, and wherein said protein has nitrite reductase activity.

It is a further object of the present invention to provide the above feed composition,wherein said microbe is characterized by enhanced expression of a gene coding for nitrite reductase.

It is a further object of the present invention to provide the above feed composition, wherein said gene coding for nitrite reductase is derived from a bacterium of the genus *Escherichia,* a coryneform bacterium, or a bacterium of the genus *Bacillus.*

It is a further object of the present invention to provide the above feed composition, wherein said gene coding for said nitrite reductase comprises a nucleotide sequence selected from the group consisting of:
(A)a nucleotide sequence selected from the group consisting of SEQ ID NO: 9, 13, and 21; and
(B)_a nucleotide sequence which is able to hybrid under stringent conditions with the nucleotide sequence selected from the group consisting of SEQ ID NO: 9, 13, and 21, or with a probe prepared from one of said nucleotide sequences, wherein said gene codes for a protein having nitrite reductase activity.

It is a further object of the present invention to provide a method of improving the growth of a ruminant by administering the above feed composition to said ruminant.

It is a further object of the present invention to provide a feed composition for ruminants comprising a microbe having nitrite reductase activity, wherein said microbe is selected from the group consisting of intestinal bacteria, coryneform bacteria, *Bacillus subtilis,* bacteria of the genus *Methylophilus, Actinomyces,* ruminal bacteria, and a combination thereof.

It is a further object of the present invention to provide the above feed composition wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20, 22, and combination thereof, except that 1 to 30 amino acids have been substituted, deleted, inserted, or added, and wherein said protein has nitrite reductase activity.

The methane production inhibitor and feed composition of the present invention significantly inhibits the production of methane in the rumen of a ruminant and enhances the energy efficiency of feed. Furthermore, by reducing the production of methane, a greenhouse gas, it contributes to the solution of environmental issues such as global warming. Still further, the methane production inhibitor of the present invention is useful in preventing the nitrite intoxication of ruminants.

### BRIEF DESCRIPTION OF THE DRAWINGS

Figure 1 shows profiles of *in vitro* cumulative CH₄ production in cultures of mixed ruminal bacteria treated with nitrate.
Figure 2 shows profiles of *in vitro* cumulative CH₄ production in cultures of mixed ruminal bacteria treated with nitrite.
Figure 3 shows profiles of *in vitro* cumulative CO₂ production in cultures of mixed ruminal bacteria treated with nitrate.
Figure 4 shows profiles of *in vitro* cumulative CO₂ production in cultures of mixed ruminal bacteria treated with nitrite.
Figure 5 shows profiles of ruminal nitrite accumulation in *in vitro* continuous incubation of mixed ruminal bacteria treated with nitrate.
Figure 6 shows profiles of ruminal nitrite accumulation in *in vitro* continuous incubation of mixed ruminal bacteria treated with nitrite.

### DETAILED DESCRIPTION OF THE INVENTION

The present invention is described in detail below.

The methane production inhibitor of the present invention is characterized by comprising a microbe-derived nitrite reductase as active ingredient and by enhancing nitrite reduction activity in the rumen. The ruminant referred to in the present invention is a mammal belonging to the suborder of ruminants of the Artiodactyla, having a stomach divided into three or four compartments, and ruminating food. Examples are cattle and sheep.

The nitrite reduction activity referred to in the present invention means activity reducing nitrites that is catalyzed by nitrite reductase. In the present invention, "enhancing nitrite reduction activity" means that the administration of a methane production inhibitor comprising nitrite reductase derived from a microbe increases nitrite reduction activity in the rumen relative to when no such inhibitor is administered. It suffices for nitrites in the rumen to be reduced to ammonia to a greater degree than when no such inhibitor is administered. Nitrite reduction activity, when given per hour per volume (L) of rumen, is desirably greater than or equal to 0.1 mM/h/L, preferably greater than or equal to 0.2 mM/h/L.

Nitrite reductase is an enzyme that catalyzes a nitrite reduction reaction. The nitrite reductase employed as an active ingredient in the present invention may either be derived from microbes originally present in the rumen, or derived from microbes not present in the rumen. It need only be a microbe-derived enzyme having nitrite reduction activity that does not greatly compromise the natural balance of the various microbe groups living symbioticallyin the digestive system of a ruminant when administered.

One or more enzyme that is derived from an intestinal bacterium such as those of microbes of the genera *Escherichia, Salmonella, Shigella,* and *Enterobacter;* a coryneform bacterium such as *Corynebacterium glutanicum;* a bacillus such as *Bacillus subtilis;* a C 1 compound-assimilating bacterium such as those of the genus *Methylophylus,* genus *Achromobacter,* genus *Pseudomonas,* genus *Protaminobacter,* genus *Methylomonas,* genus *Methylobacillus* (Patent Application Publication No. Heisei 4-91793), and genus *Bacillus* (Patent Application Publication No. Heisei 3-505284); Actinomyces such as *Streptomyces coelicolor;* a ruminal bacterium such as *Pyrobaculum aerophilum, Selenomonas ruminantium, Veillonella parvula,* and *Wolnella succinogenes;* may be employed as the nitrite reducing enzyme of the present invention.

Enzymes derived from intestinal bacterium includes the enzymes from the bacterium belonging to the genera *Enterobacter, Erwinia, Escherichia, Klebsiella, Pantoea, Providencia, Salmonella, Serratia, Shigella, Morganella* etc. Specifically, those classified into the *Enterobacteriaceae* according to the taxonomy used in the NCBI (National Center for Biotechnology Information) database
(http://www.ncbi.nlm.nih.gov/htbin-post/Taxonomy/wgetorg?mode=Tree&id=1236&lvl= 3&keep=l&srchmode=l&unlock) can be used. A bacterium belonging to the genus of *Escherichia* is preferred.

Enzymes derived from the bacterium belonging to the genus of Escherichia particularly nontoxic strains K and B (for example, strain W3110 of strain K-12, which is a derivative of the K strain and strain MG1655) reported in Neidhardt et al. (Neidhardt, F.C. et al., Escherichia coli and Salmonella Typhimurium, American Society for Microbiology, Washington D.C., 1208, Table 1), can be utilized. Examples of wild-type strains of Escherichia coli include, but are not limited to, the K12 strain and derivatives thereof, Escherichia coli MG1655 strain (ATCC No. 47076), and W3110 strain (ATCC No. 27325). These strains are available from the American Type Culture Collection (ATCC, Address: P.O. Box 1549, Manassas, VA 20108, United States of America).

The term "Bacillus bacterium" means that the bacterium classified as the genus Bacillus according to the classification known to a person skilled in the art of microbiology.

In the present invention, the enzyme from Bacillus bacterium include, but are not limited to, the Bacillus subtilis 168 Marburg strain (ATCC 6051), Bacillus subtilis PY79 strain (Plasmid, 1984, 12, 1-9) and so forth, and examples of Bacillus amyloliquefaciens include, but are not limited to, Bacillus amyloliquefaciens T strain (ATCC 23842), Bacillus amyloliquefaciens N strain (ATCC 23845) and so forth.

Examples of coryneform bacteria are microbes belonging to the genus *Corynebacterium,* microbes belonging to the genus *Brevibacterium,* and microbes belonging to the genus *Arthrobacter* (Int. J. Syst. Bacteriol., 41, 255 (1991)). Of these, desirable examples are those belonging to the genera *Corynebacterium* and *Brevibacterium.* Preferred examples are enzymes derived from microbes belonging to *Corynebacterium glutamicum(ATCC13032* ATCC13869), *Brevibacterium* flavum(ATCC13826,14067), *Brevibacterium ammoniagenes(ATCC6871,6872),* and *Brevibacterium lactofermentum(ATCC13869).*

Example of ruminal bacteria are microbes belonging to *Anaerovibrio , Butyrivibrio Bacteroides, Clostridium, Eubacterium, Lachnospire, Megasparea,,Metahnobrevibacter, Methanosarcina,Ruminococcus, Selenomonas Selenomonas Streptococcus, Succinivibrio, Wolinella* (nutritional physiology of ruminant;Rural Culture Association 1998).

Enzymes derived from, for example, AS1 strain (NCIMB10515) of *Methylophilus methylotrophus,* and *Bacillus* methanolicus, are preferable examples of those derived from C1 compound-assimilating microbes.

The nitrite reductase activity of these enzymes can be measured and confirmed by observing the reduction of nitrites when the enzymes are added to a suitable reaction or bacterial culture system. Furthermore, the activity of those employing ferredoxin as an electron donor (EC 1.7.2.2, EC 1.7.7.1) can be measured by the method of Okunuki et al. (1970, Cytochrome p. 195-207) for EC 1.7.2.2 and the method of Ida et al. (1976, Tanpakushitsu, Kakusan, Koso, special edition, p. 349-355) for EC 1.7.7.1.. The activity of those employing NAD(P)H as electron donor (EC 1.7.1.4) can be measured by the method of N. R. Harbourne (Molecular Microbiology, 6, 2805 (1992)).

A nitrite reductase that has been registered in a database (Swiss-Prot, EMBL) may be employed in the present invention.

For example, the following enzymes that have been registered as EC 1.7.1.4 nitrite reductases may be employed; however, the present invention is not limited thereto.
1. sp: NASD_BACSU [P42435] Nitrite reductase [NAD(P)H] (derived from *Bacillus subtilis).*
2. sp: NASE_BACSU [P42436] Assimilatory nitrate reductase [NAD(P)H] small subunit (derived from *Bacillus subtilis).*
3. sp: NASB_BACSU Assimilatory nitrate reductase electron transfer subunit.
4. sp: NIRB_ECOLI [P08201] Nitrate reductase [NAD(P)H] large subunit (derived from *E. coli)* (SEQ ID NO: 10).
5. sp: NIRD_ECOLI [P23675] Nitrite reductase [NAD(P)H] small subunit (derived from *E. coli)* (SEQ ID NO: 12)

For example, the following enzymes, categorized as EC 1.7.2.2 nitrite reductases, may be employed.
1. sp: NRFA_ECOLI [P32050] Cytochrome c-552 precursor (Ammonia-forming cytochrome c nitrite reductase) (Cytochrome c nitrite reductase) (derived from E. *coli)* (SEQ ID NO: 14).
2. prf: 2001439E *nrf*B protein - (derived from *E. coli)* (SEQ ID NO: 15)
3. prf: 1924370B *Nrf*C protein - (derived from *E. coli)* (SEQ ID NO: 16)
4. prf: 1924370C *Nrf*D protein - (derived from *E. coli)* (SEQ ID NO: 17)
5. prf: 2001439EL *nrf*E protein - (derived from *E. coli)* (SEQ ID NO: 18)
6. sp: NRFG_ECOLI [P32711] Formate-dependent nitrite reductase complex *nrf*F subunit precursor (derived from *E. coli)* (SEQ ID NO: 19)
7. sp: NRFG_ECOLI [P32712] Formate-dependent nitrite reductase complex *nrf*G subunit (derived from *E. coli)* (SEQ ID NO: 20)

For example, the following enzymes, categorized as EC 1.7.7.1 nitrite reductases, may be employed.
1. NP_602008 putative nitrite reductase (derived from *Corynebacterium glutamicum)* (SEQ ID NO: 22)
2. BAC 19454 putative ferredoxin-nitrite reductase (derived from *Corynebacterium efficies)*
3. CAC33947 putative nitrite sulphite reductase (derived from *Streptomyces coelicolor)*

Many nitrite reductases have a structure comprising multiple subunits. However, some of the subunits may be missing so long as nitrite reduction activity is enhanced in the rumen. To the extent that the nitrite reductase activity of the protein is not lost, the nitrite reductase of the present invention may be anything coding for a nitrite reductase comprising one or several amino acid substitutions, deletions, insertions, additions, or inversion at one or several sites. Here, the term "several" varies with the type and position of the amino acid residues within the three-dimensional structure of the protein, but specifically refers to from 2 to 30, preferably from 2 to 20, and more preferably from 2 to 10 amino acids.

The nitrite reductase of the present invention includes not only enzymes derived from the above-described microbes inherently having nitrite reducing activity, but also enzymes derived from microbes which inherently have no nitrite reductase activity but have acquired ability to reduce nitrites through an introduction of a gene coding for nitrite reductase. Furthermore, the nitrite reductases of the present invention may be derived from wild-type strains of microbes having nitrite reduction capability, but are desirably derived from microbes characterized by having been modified so that intracellular nitrite reductase activity is enhanced.

In the present invention, the phrase "modified so that intracellular nitrite reductase activity is enhanced" means modified to heighten the nitrite reductase activity per cell. This also includes the case where nitrite reductase activity has increased beyond that of the unmodified strain, such as a wild-type strain, and preferably not less than 1.5-fold, more preferably not less than 2-fold, and most preferably not less than 3-fold of a wild-type or non-modified strain. For example, this corresponds to the case where the number of molecules of nitrite reductase per cell has been increased and the case where the activity per nitrite reductase molecule has been increased. Examples of wild-type strains for comparison are E. *coli* MG1655 and *Bacillus subtilis* 168.

Various methods are known for increasing nitrite reductase activity. One method of enhancing enzymatic activity is to increase the expression level of the gene coding for the enzyme. Known methods include the method of increasing the number of the gene copies in extrachromosomal DNA such as plasmids (see U.S. Patent No. 5,830,716), the method of increasing the number of the gene copies on the chromosomes, the method of introducing a mutation into the promoter of the gene coding for the enzyme to increase activity (see International Publication WO 00/18935), and the method of substituting the promoter with a stronger promoter. Nitrite reductase activity can also be enhanced by introducing a mutation into the coding region of the gene to heighten the specific activity of the nitrite reductase (see Cole J., FEMS Microbiology Letters Volume 136, February 1996, Issue 1). Here, the gene employed may be either a gene coding for the inherent nitrite reductase in a microbe to be modified, or a gene coding for that of some other microbe.

Examples of genes coding for nitrite reductases are the *nir*D and *nir*B genes for enzyme (EC 1093349888125_0.4) employing NAD(P)H as an electron donor and the *nrf*ABCDEFG gene (EC 1.7.2.2) or *nir*A gene (EC 1.7.7.1) for enzyme employing ferredoxin as an electron donor. However, any gene coding for a nitrite reductase that can be expressed in a host may be employed. The gene coding for nitrite reductase may be a native gene or may be a gene derived from some other organism. A portion of the gene may be deleted so long as the gene has nitrite reductase activity. The following sequences, which have already been disclosed in GenBank or the like, may be employed as the gene coding for nitrite reductase.

The following genes and microbes are examples of genes and microbes having nitrite reductase employing NAD(P)H as an electron donor (EC: 1093349888125_1.4), and the protein coded for by the gene may be employed as the nitrite reductase of the present invention.

### E. coli

GenBank Accession Number: AAC76390 (*nir*B) nitrite reductase (NAD(P)H) subunit [gi: 1789765] (SEQ ID NO: 9 (nucleotide nos. 1 to 2544), amino acid SEQ ID NO: 10)
GenBank Accession Number: AAC76391 *(nir*D*)* nitrite reductase (NAD(P)H) subunit [gi: 1789766] (SEQ ID NO: 11 (SEQ ID NO: 9: nucleotide sequence nos. 2,541 to 2,864), amino acid SEQ ID NO: 12)

### Salmonella typhimurium CT18 GB:

GenBank Accession Number: CAD08139 (*nir*B) nitrite reductase (NAD(P)H) large subunit [gi: 16505117]
GenBank Accession Number: CAD08138 (*nir*D) nitrite reductase (NAD(P)H) small subunit [gi: 16505116]

### Salmonella typhimurium GB:

GenBank Accession Number: AAL22336 (*nir*B) nitrite reductase large subunit [gi: 16422031]
GenBank Accession Number: AAL22337 (*nir*D) nitrite reductase small subunit [gi: 16422032]

### Shigella flexneri GB:

GenBank Accession Number: AAP19331 *(nir*B*)* nitrite reductase (NAD(P)H) subunit [gi: 30043611] AAN44848 (*nir*D) nitrite reductase (NAD(P)H) subunit [gi: 24053832]

### Bacillus subtilis GB:

GenBank Accession Number: CAB12123 (*nas*E) assimilatory nitrite reductase (subunit) [gi: 2632615]
GenBank Accession Number: CAB 12124 (*nas*D) assimilatory nitrite reductase (subunit) [gi: 2632616]
GenBank Accession Number: CAB12126 (*nas*B) assimilatory nitrite reductase (electron transfer subunit) [gi: 2632618]

Desirably, the *nir*D or *nir*B gene of *E. coli* or the *nas*D*, nas*E*,* or *nas*B gene of *Bacillus subtilis* is employed. Many of the genes coding for nitrite reductase that are denoted by EC 1.7.1.4 have an operon structure. The protein that *nir*B codes for constitutes a large subunit of nitrite reductase and the protein that *nir*D codes for constitutes a small subunit of nitrite reductase. The *nir*B gene corresponds to nucleotide numbers 1 to 2544 in SEQ ID NO: 9 and the *nir*D gene corresponds to nucleotide sequence numbers 2541 to 2864 (SEQ ID NO: 11) in SEQ ID NO: 9.

Examples of genes or microbes having enzymes employing ferredoxin as an electron donor (EC 1.7.2.2) are given below. These genes are thought to code for various subunits of nitrite reductases, and the proteins coded for by the genes can be used as the nitrite reductase of the present invention (Mol. Microbiol. Apr. 1994: 12(1):

### 153-63)(nrfABCDEFG).

*E. coli nrf* operon (SEQ ID NO: 13)
AAC77040 (*nrf*A) periplasmic cytochrome [gi: 1790506] (SEQ ID NO: 13, nucleotide nos. 303-1739; amino acid SEQ ID NO: 14)
AAC77041 (*nrf*B) a penta-haeme cytochrome c [gi: 1790507] (SEQ ID NO: 13, nucleotide nos. 1784-2350; amino acid SEQ ID NO: 15
AAC77042 (*nrf*C) Fe-S centers [gi: 2367345] (SEQ ID NO: 13, nucleotide nos. 2347-3018; amino acid SEQ ID NO: 16)
AAC77043 (*nrf*D) transmembrane protein; [gi: 1790509] (SEQ ID NO: 13, nucleotide nos 3015-3971; amino acid SEQ ID NO: 17)
AAD13457 (*nrf*E) possible assembly function; [gi: 1790511] (SEQ ID NO: 13, nucleotide nos 4051-5709; amino acid SEQ ID NO: 18)
AAD13458 (*nrf*F) involved in attachment of haem c to cytochrome c552 [gi: 1790512] (SEQ ID NO: 13, nucleotide nos. 5702-6085; amino acid SEQ ID NO: 19)
AAD13459 (*nrf*G) [gi: 1790513] (SEQ ID NO: 13, nucleotide nos 6082-6678; amino acid SEQ ID NO: 20)

Examples of genes or microbes having enzymes employing ferredoxin as electron donor (EC 1.7.7.1) are given below. The proteins coded for by the genes can be used as the nitrite reductase of the present invention.

### (nirA)

*Corynebacterium glutamicum* NP_602008 (*nir*A) putative nitrite reductase [gi: 19554006] (SEQ ID NO: 21)
Mycobacterium tuberculosis CAA17319 (*nir*A) possible oxidoreductase [gi: 16505265]
Streptomyces coelicolor CAC33947 (*nir*A) putative nitrite/sulphite reductase [gi: 13276829]
Pyrobaculum aerophilum AAL64294 (*nir*A) ferredoxin-nitrite reductase [gi: 18160980]
   A homologue of the *nir*A gene of *Corynebacterium glutamicum* is employed with preference.

The genes coding for these nitrite reductases can be obtained by the methods described below. For example, they can be obtained by polymerase chain reaction (PCR: see White, T. J. et al., Trends Genet. 5, 185 (1989)) employing *E. coli* chromosomal DNA as template. The oligonucleotides having the nucleotide sequences shown in SEQ ID NOS: 1 and 2 are examples of amplification primers for the *nir*BD coding for the nitrite reductase of bacteria of the genus *Escherichia.* Examples of sources of such chromosomal DNA are, when obtaining *nir*BD and *nrf*ABCDEFG, wild-type strains of E. *coli* such as strain W3110 (ATCC39936) and strain MG1655, and a wild-type strain 168 of Bacillus subtilis. When obtaining *nir*A homologues, an example is *Corynebacterium glutamicum* ATCC13869.

Strains W3110, MG1655, 168, and ATCC13869 are available from the American Type Culture Collection, 10801 University Boulevard, Manassas, VA 20110-2209, USA.

So long as the nitrite reductase activity is not lost, the nitrite reductase of the present invention may contain one or several amino acid substitutions, deletions, insertions, additions, or inversions at one or several sites. Here, the term "several" varies with the type and position of the amino acid residues within the three-dimensional structure of the protein, but specifically refers to from 2 to 30, preferably from 2 to 20, and more preferably from 2 to 10 amino acids.

DNA coding for substantially the same protein as the above-described nitrite reductases can be obtained by, for example, modifying the coding region of the gene for a nitrite reductase by a site-specific mutagenesis method so that the amino acid sequence denoted by SEQ ID NO: 10, 12, 14 to 20, or 22 contains from 1 to 30 amino acid substitutions, deletions, additions, or inversions. DNA modified in this manner may also be obtained by conventional mutation treatments. Examples of mutation treatments are methods by which a DNA is treated in vitro with hydroxylamine or the like, and methods by which a microbe such as a bacterium of the genus *Escherichia* having a DNA prior to mutation treatment is treated with a conventionally employed mutating agent such as ultraviolet radiation, N-methyl-N'-nitro-N-nitrosoguanidine (NTG), or EMS.

Mutation of the gene coding for the above nitrite reductase may be a conservative mutation in which the activity of the nitrite reductase is conserved. The above substitution is a change in which one or more residues in the amino acid sequence are removed and another residue is inserted at that site. Examples of the substitution of one or more original amino acids of a nitrite reductase with one or more amino acids in what is considered to be a conservative substitution are: substituting Ser or Thr for Ala; substituting Gln, His, or Lys for Arg; substituting Glu, Gln, Lys, His, or Asp for Asn; substituting Asn, Glu, or Gln for Asp; substituting Ser or Ala for Cys; substituting Asn, Glu, Lys, His, Asp, or Arg for Gln; substituting Asn, Gln, Lys, or Asp for Glu; substituting Pro for Gly; substituting Asn, Lys, Gln, Arg, or Tyr for His; substituting Leu, Met, Val, or Phe for Ile, substituting Ile, Met, Val, or Phe for Leu; substituting Asn, Glu, Gln, His, or Arg for Lys, substituting Ile, Leu, Val, or Phe for Met; substituting Trp, Tyr, Met, Ile, or Leu for Phe; substituting Thr or Ala for Ser; substituting Ser or Ala for Thr; substituting Phe or Tyr for Trp, substituting His, Phe, or Trp for Tyr; and substituting Met, Ile, or Leu for Val.

DNA having the mutation as set forth above is expressed in a suitable cell and the activity of the expression product is tested to obtain a DNA coding for a protein substantially identical to the nitrite reductase.

The gene coding for the nitrite reductase employed in the present invention includes DNA coding for a protein having nitrite reductase activity that hybridizes under stringent conditions with a nucleotide sequence of SEQ ID NOS: 9, 13, or 21, or with a probe prepared from the nucleotide sequence. The term "stringent conditions" as used herein refers to conditions under which a specific hybrid forms and nonspecific hybrids do not form. The reduction of such conditions to numerical values is difficult, but as an example, they are conditions under which DNA strands with a high degree of homology, for example, greater than or equal to 80 percent, preferably greater than or equal to 90 percent, and more preferably, greater than or equal to 95 percent, hybridize, and DNA strands with a lower degree of homology do not hybridize. As a further example, they are the usual Southern hybridization washing once or preferably 2-3 times under 60°C at a salt concentration corresponding to 1 x SSC, 0.1 percent SDS, preferably 0.1 x SSC, 0.1 percent SDS.

A portion of the nucleotide sequence of SEQ ID NO: 9, 13, or 21 can be employed as probe, for example. Such a probe can be prepared employing an oligonucleotide prepared based on the nucleotide sequence of SEQ ID NO: 9, 13, or 21 as primer and conducting PCR with a DNA fragment containing the nucleotide sequence of SEQ ID NO: 1 or 3 as template. When a DNA fragment of about 300 bp in length is employed as probe, an example of the hybridization washing conditions is 50°C, 2 x SSC, and 0.1 percent SDS.

A specific example of a DNA coding for a protein substantially identical to a nitrite reductase is a DNA preferably having a degree of homology of greater than or equal to 70 percent, preferably greater than or equal to 80 percent, more preferably greater than or equal to 90 percent, and still more preferably greater than or equal to 95 percent with the amino acid sequence given by SEQ ID NO: 10, 12, 14 to 20, or 22, as well as coding for a protein having activity equivalent to that of a nitrite reductase. Any DNA having nitrite reductase activity will suffice.

Genes coding for nitrite reductase of other microbes may be obtained by PCR or the like from the chromosomal DNA of the microbe in the same manner as a well-known method used to obtain a gene.

Chromosomal DNA may be prepared by the method of Saito and Miura (see H. Saito and K. Miura, Biochem. Biophys. Acta, 72, 619 (1963), Book of Biological Experiments, compiled by the Japan Bioengineering Society, pp. 97-98, Baifukan, 1992) from microbes that are DNA donors.

The obtained gene is inserted into vector DNA that can be autonomously replicated in E. *coli* and/or the cell of the targeted microbe to prepare recombinant DNA. When this is incorporated into *E. coli,* the subsequent operations are facilitated. Examples of vectors that can be autonomously replicated in E. *coli* are pSTV29, pUC19, pUC18, pHSG299, pHSG399, pHSG398, RSF1010, pBR322, pACYC184, and pMW219. The use of an expression vector with a His-Tag or the like to permit purification by large scale expression of a protein in the cell is also possible.

To ligate the obtained gene with a vector functioning in the target cell to prepare recombinant DNA, it suffices to cleave the vector with a restriction enzyme which recognizes the same sequence as the terminal of the above gene and employ a ligase such as T4 DNA ligase to bind the gene to the vector.

Nitrite reductase activity is enhanced by enhancing expression of the gene coding for the protein constituting the nitrite reductase, as set forth above. The level of expression of a single gene is enhanced by increasing the number of copies of the gene in the manner set forth above, for example. By way of example, it suffices to link the above gene fragment to a vector functioning within a microbe, preferably a multicopy-type vector, to prepare recombinant DNA, and introduce this into a host to transform the host.

Transformation methods that have been reported thus far may be employed to introduce the recombinant DNA into a microbe. For example, as reported for E. *coli* strain K-12, there exists the method of treating a recipient microbial cell with calcium chloride to increase DNA permeability (Mandel, M. and Higa, A., J. Mol. Biol., 53, 159 (1970)), and as reported for *Bacillus subtilis,* there exists the method of preparing competent cells from cells in the growth stage to introduce DNA (Duncan, C.H., Wilson, G.A., and Young, F.E., Gene, 1, 153 (1977)). Alternatively, it is possible to apply the method, known for *Bacillus subtilis,* Actinomyces, and yeast, of converting the cells of a DNA recipient microbe into protoplast or spheroplast form, which readily incorporate recombinant DNA, and introducing the recombinant DNA into a DNA recipient microbe (Chang, S. and Choen, S.N., Molec. Gen. Genet., 168, 111 (1979)); Bibb, M.J., Ward, J.M. and Hopwood, O.A., Nature, 274, 398 (1978); Hinnen, A., Hicks, J.B., and Fink, G.R., Proce. Natl. Acad. Sci. USA, 75 1929 (1978)). Microbe transformation is also possible by the electric pulse method (Japanese Patent Application Publication No. Heisei 2-207791).

The number of copies of a gene can also be increased by introducing multiple copies of a gene onto the chromosomal. DNA of a microbe. Multiple copies of a gene can be introduced onto the chromosomal DNA of a microbe by employing as target a sequence which are present on chromosomal DNA in multiple copies, and conducting homologous recombination. Repetitive DNA, inverted repeats present at the end of transposon, and the like can be employed as sequences which are present on chromosomal DNA in multiple copies. Alternatively, as disclosed in Japanese Patent Application Publication No. Heisei 2-109985, a target gene can be loaded onto a transposon and transferred to introduce multiple copies onto chromosomal DNA.

In addition to the above-described genetic amplification, nitrite reductase activity can also be enhanced by substituting an expression control sequence, such as a promoter, coding for a nitrite reductase on chromosomal DNA or a plasmid with a stronger expression control sequence. For example, lac promoter, tac promoter, trp promoter, and trc promoter are known as strong promoters. Furthermore, as disclosed in International Application Publication No. WO00/18935, a substitution of several nucleotides can be introduced into a promoter region of a gene to render it stronger. These promoter substitutions or modifications enhance the expression of the gene coding for the nitrite reductase, enhancing nitrite reductase activity. These substitutions and modifications in the expression control sequence may also be combined with an increase in the number of copies of the gene.

The expression of many genes coding for nitrite reductases is regulated under aerobic conditions. By substituting the promoter upstream from the gene coding for a nitrite reductase with a constitutively expressed strong promoter that is not regulated by aerobic conditions, it is possible to increase the level of expression independently of aeration conditions. For example, the substitution of the control region with a tac promoter or the like is conceivable. Furthermore, the incorporation of site-specific mutations so that the control region no longer functions permits an increase in the level of expression that is independent of ventilation conditions.

The usual microbe culturing methods, such as liquid culturing and solid culturing, can be employed to culture a microbe. For reasons of economy, methods of liquid culturing under aerobic conditions are desirable. Examples are the shaking culture method and the aerated stirring culture method under aerobic conditions. The expression of genes coding for nitrate reductase is often regulated under aerobic conditions. When expression is regulated, the method of avoiding contact with air using the paraffin method under anaerobic conditions, the method of adding a reducing agent to the medium, and the method of backfilling a sealed container with nitrogen gas may be employed.

Any culture components that allow the strain being used in the present invention to proliferate may be employed. For example, a culture containing a carbon source in the form of an assimilatable carbon compound, or a substance containing such a compound, can be employed; glucose, starches, starch hydrolysates such as liquid starch, and sugars such as molasses may be employed singly or in combination. As a further example, a nitrogen source in the form of an assimilatable compound, or a substance containing such a compound, can be employed; organic nitrogen-containing compounds including various amino acids, corn steep liquor, malt extract, peptones, soybean meal, defatted soybean meal, and inorganic nitrogen compounds including ammonium chloride, ammonium sulfate, and other ammonium salts may be employed singly or in combination. Still further, various organic and inorganic substances, as well as materials containing the same, that are necessary for the growth of microbes and the production of enzymes may be added. These include salts such as phosphates, magnesium salt, calcium salt, and manganese salt, as well as vitamins and yeast extracts.

The microbe-derived nitrite reductase of the present invention may be a nitrite reductase that is purified from the culture solution of a microbe, for example. Nitrite reductase may be purified by ammonium sulfate precipitation, column chromatography, ethanol precipitation, or the like. So long as the microbe of the microbe-derived nitrite reductase of the present invention exhibits nitrite reduction activity, it may be provided in any of a variety of forms. For example, it may be derived from the culture solution of the microbe by drying the microbe mass itself, be in the form of a microbe culture solution product obtained by treatment with acetone or the like, or be in the form of culture solution containing the microbe.

The nitrite reductase of the present invention may be prepared by culturing the above-described microbe in a culture medium and, during a period where protein purification is easy, such as during the exponential growth phase, purifying the enzyme by ammonium sulfate precipitation, column chromatography, ethanol precipitation, or the like. The unrefined culture solution in liquid form or the dried microbe mass may be employed as a methane production inhibitor containing nitrite reductase. The enzyme or enzyme solution employed in the present invention can be produced using culture components consistent with the strain employed and the enzyme being targeted. When employed in an enzyme solution, it is effective to continue treatment to obtain a suitably concentrated form.

In the present invention, enhanced nitrite reductase activity in the rumen can be confirmed using an artificial rumen system (T. Hino et al., J. Gen. Appl. Microbiol., 39, 35-45 (1993) or confirmed in vivo by actual oral administration to a ruminant.The in vitro rumen simulation technique (RUSITEC), described by Czerkawski and Breckenridge (1977) can be used as an artificial rumen system (product of Sunshinkogyo
http://www.sanshinkogyo.co.jp/RusitecS.htm)),

The methane production inhibitor of the present invention may be administered to ruminants in a variety of forms, such as powders, grains, and tablets. Excipients, extenders, and the like may be suitably added as needed. The proportion of the nitrite reductase of the present invention in the methane production inhibitor may be determined based on the goal for which it is being used, or the like. When protein purity is high or specific activity is high, the nitrite reductase is administered in a low proportion, and when administering the culture medium itself or when specific activity is low, the nitrate reductase is administered in a high proportion. Administration may be directly into the rumen fluid of a ruminant, or a method of oral administration may be employed, for example.

The methane production inhibitor of the present invention can be administered at any time when feed is present within the rumen; there is no specific limitation. However, since it is desirable for the methane production inhibitor to be present in the rumen during methane generation, it is desirable for the methane production inhibitor to be administered immediately before or simultaneously with feed administration. Particularly effective administration is possible by blending the methane production inhibitor into the feed.

The administration amount of methane production inhibitor of the present invention is not specifically limited. For example, administration level of nitrite reductase which enhances nitrite reduction activity within the rumen may be 0.1 mM or more of the nitrite reduction activity per hour per volume (L) of rumen (0.1 mM/h/L or more), preferably 0.2 mM/h/L or more. The volume of the rumen can be converted into body weight based on the average volume of the rumen and the average body weight. Suitable adjustment is made for the microbe being employed and the animal receiving the administration.

The feed composition for ruminants of the present invention is a feed composition in which the above-described methane production inhibitor is added to a conventional feed composition. The blending proportion of the methane production inhibitor in the feed composition is normally from 0.1 to 10 weight percent, preferably from 1 to 5 weight percent. The conventional feed composition for ruminants is not specifically limited; a commercial product may be employed without alteration, or, as needed, suitable silage or hay may be added to a commercial product. An example of a bovine feed composition is 40 percent corn silage, 14 percent Sudan hay or alfalfa hay cubes, and 46 percent of a commercial blend of feed. An example of a commercial blend of feed contains not less than 16 percent of crude protein and not less than 71 percent digestible total nutrients.

An example of an ovine feed composition contains 85 percent timothy hay and 15 percent of a commercial blend of feed. In the present invention, the feed composition in which a methane production inhibitor has been blended may be freely given to the ruminant. It may also be given for extended periods.

Administering the feed composition containing a methane production inhibitor of the present invention inhibits methane production in the rumen of ruminants and enhances feed efficiency. Furthermore, the growth of ruminants is enhanced and nitrite intoxication of ruminants can be prevented.

The present invention is specifically described below through Examples. However, the present invention is not limited to the Examples given below.To a system in which nitrites and nitrates had been suitably added to rumen fluid in vitro, *E. coli* with enhanced nitrate reduction activity or a wild-type strain *E. coli* was added to show how nitrites were rapidly reduced and the generation of methane was inhibited.

### Example 1

### Construction of Eshcerichia bacterium which has an enhanced nitrite reduction activity

### <Construction of E. coli nir-Ptac strain which has an enhanced nitrite reduction activity>

*E. coli nir*-P*tac* strain has nitrite reductase gene *nir*BD*,* the native promoter of which is replaced with tac promoter. This strain was constructed as follows: a DNA fragment containing *nir*BD genes was amplified by PCR with *Pyrobest* DNA polymerase (Takara Shuzo Co., Ltd., Shiga, Japan), using W3110 genomes as a template, and ni5 (SEQ ID NO: 1/ TCA GCC GTC ACC GTC AGC ATA ACA C) and nic4.1 (SEQ ID NO: 2/ CCG ACA GGC GTG CAA TGC GCG CAG C) as primers. PCR was conducted with 30 cycles of 10 seconds at 98°C, 30 seconds at 59°C and 3.5 minutes at 72°C. The amplified fragment was inserted into the *Sma*I site of pHSG398 vector (Takara Bio Co., Ltd.) and checked to have no PCR error. The constructed plasmid was designated pNIRBD. Plasmid pMW219-*nir*BD was constructed by cutting *nir*BD genes off pNIRBD with *Kpn*I and *Xba*I, and inserting the *nir*BD genes into the pMW219 (Nippon Gene) at *Kpn*I and *Xba*I sites. This plasmid has *nir*BD operon in the same direction as lacZ gene.

Another PCR with *Pyrobest* DNA polymerase (Takara Bio Co., Ltd.) was performed using pMW219-*nir*BD as a template, and nirex5 (SEQ ID NO: 3/ AAA AGA ATT CGA GGC AAA AAT GAG CAA AGT) and nirex3 (SEQ ID NO: 4/ CCC CAA GCT TCA TGC AAA AAG GGG AGG CAT) as primers. PCR was conducted with 30 cycles of 10 seconds at 98°C, 30 seconds at 57°C and 3.5 minutes at 72°C. The amplified fragment was cut off with *Eco*RI and *Hind*III and inserted into the pKK223-3 expression vector (Amersham Pharmacia Biotech) at the *Eco*RI and *Hind*III sites. The inserted fragment was checked and found to have no PCR error. The constructed plasmid was designated pKK-nirEx.

A DNA fragment with the *tac* promoter used for gene expression studies in *E. coli* (Xue et al., 1996, Temperature-regulated expression of the *tac*/*Lacl* system for overproduction of a fungal xylanase in *Escherichia coli.* Appl. Microbiol. Biotechnol. 45, 120-126) and a 5' portion of the *nir*BD genes was amplified by PCR with *Pyrobest* DNA polymerase (Takara Bio Co., Ltd.), using pKK-nirEx as a template, and pKK-c200 (SEQ ID NO: 5/ CGG GGT ACC TTC TGG CGT CAG GCA GCC AT) and NI'-c2035 (SEQ ID NO: 6/ ACA TGC ATG CCG TCT ACG CCC AGC AGT TTC) as primers. PCR was conducted with 30 cycles of 10 seconds at 98°C, 30 seconds at 57°C and 1 minutes at 72°C. The fragment amplified was digested with *Kpn*I and *Sph*I and designated frag.1.

A DNA fragment containing the upstream region of *nir*BD was amplified by PCR with *Pyrobest* DNA polymerase (Takara Bio Co., Ltd.), using W3110 genome as a template, and NI'-1 (SEQ ID NO: 7/ CGG AAT TCG TAT GAA GGG CGT CAG CGC G) and NI'-c925 (SEQ ID NO: 8/ CGG GGT ACC TTC TTA AGT CAC GGA ATT GT) as primers. PCR was conducted with 30 cycles of 10 seconds at 98°C, 30 seconds at 57°C and 1 minute at 72°C. The fragment amplified was digested with *Kpn*I and *Eco*RI and designated frag.2.

pHSG299 vector (Takara Bio Co., Ltd.) was digested with *Eco*RI and *Sph*I and ligated with frag.1 and frag.2 constructed above. A clone which has one copy of frag.1 located at *Eco*RI end and one copy of frag.2 located at *Sph*I end of the digested pHSG299 was chosen. The inserted fragment was checked to have no PCR error. The constructed plasmid was designated pHSG*-nir-*P*tac.*

The inserted fragment of pHSG*-nir-*P*tac* was cut out with *Hind*III and ligated into *Hind*III sites of temperature-sensitive vector, pMAN997 (International publication pamphlet WO99/03988). The resulting plasmid was designated pMAN-*nir*-P*tac*. *Escherichia coli* strain W3110 was transformed with pMAN*-nir-*P*tac* according to a method of C. T. Chung *et al.* and cultured at 30°C on LB + ampicillin plate. Colonies which appeared on the plate were selected and cultured overnight at 30°C. The obtained culture was diluted to 10⁻³, spread onto LB + ampicillin plate and cultured at 42°C. Colonies which appeared on the plate were selected and spread onto LB + ampicillin plate and cultured at 30°C. Colonies which appeared on a 1/8 part of the plate were suspended in 2 ml of LB medium and cultured with shaking for 4-5 hours at 42°C. The obtained culture was diluted to 10⁻⁵ and spread onto LB plate. A few hundred of the obtained colonies were seeded onto an LB plate and an LB + ampicillin plate. Ampicillin-sensitive strains were selected by comparing growth on the both plates. The ampicillin-sensitive strains were subject to colony PCR for selecting clones with the promoter region of *nir*BD replaced by *tac* promoter through the twice recombination between genome and plasmid. The selected clone was checked to have no vector region on its genome and designated nir-Ptac.
ni5 : tca gcc gtc acc gtc agc ata aca c (SEQ ID NO: 1)
nic4.1 : ccg aca ggc gtg caa tgc gcg cag c (SEQ ID NO: 2)
nirex5 : aaa aga att cga ggc aaa aat gag caa agt (SEQ ID NO: 3)
nirex3 : ccc caa gct tca tgc aaa aag ggg agg cat (SEQ ID NO: 4)
pKK-c200 : cgg ggt acc ttc tgg cgt cag gca gcc at (SEQ ID NO: 5)
NI'-c2035 : aca tgc atg ccg tct acg ccc agc agt ttc (SEQ ID NO: 6)
NI'-1 : cgg aat tcg tat gaa ggg cgt cag cgc g (SEQ ID NO: 7)
NI'-c925 : cgg ggt acc ttc tta agt cac gga att gt (SEQ ID NO: 8)

### <Analysis of Nitrate and nitrite concentration>

Nitrate and nitrite concentration in culture supernatant was roughly estimated using NO₂₋test paper (Merck: Merckoquant Nitrite Test 1. 10022.0001, 1.10007.0001) and NO₃⁻ test paper (Merck: Merckoquant Nitrate Test 1. 10020.0001), respectively. Then, the sample was diluted to fit the nitrate and nitrite concentration between 0 and 100µM which is the measurement range of the nitrate/nitrite Assay Kit-C (Colorimetric, Dojindo) and subject to the more accurate concentration measurement with the kit. The absorption coefficient was measured using the microplate reader SPECTRA MAX 190 (Molecular Devices).

### Example 2

### Effect in in vitro rumen system

### <Materials and experimental tools>

The *in vitro* continuous incubation systems consisting of four 1000-ml fermenter vessels equipped with a buffer and solid feed input, a thermister probe, an input for nitrogen gas, magnetically stirring and near-infrared CH₄ and CO₂ analyzer (Takasugi Seisakusho Co. Ltd. Tokyo, Japan) were used in the Examples. Two nonlactating Holstein cows (average 800kg BW) used as donor cows were fitted with ruminal fistulae according to surgical procedures approved by the Institutional Animal Care and Use Committee of Obihiro University of Agriculture and Veterinary Medicine, and was housed in a tie-stall barn. The cows received a basal diet of hay (DM (dry matter): 87.33%, OM (organic matter): 98.98%, CP (crude protein): 14%, ADF (acid detergent fiber): 38.84%, NDF (neutral detergent fiber): 73.26%, ADL (acid detergent lignin): 4.10%, GE (gross energy): 4.45 Mcal) at maintenance level (55g DM kg^{0.75} BW/day) in two equal portions at 08:00 and 17:00h with free access to *ad libitum* water and block sodium chloride (Fe: 1232, Cu: 150, Co: 25, Zn: 500, I: 50, Se: 15 and Na: 382 mg/kg). Rumen fluid was collected from the cows before morning feeding and strained immediately through woven nylon cloth into Erlenmeyer flask with O₂-free headspace. One volume of strained rumen fluid (400ml) was mixed with one volume of autoclaved buffer solution (400ml) (Takahashi, J. *et al.,* Inhibitory effects of sulphur compounds, copper and tungsten on nitrate reduction by mixed rumen micro-organisms. Br. J. Nutr. 61, 741-748). All incubations were carried out anaerobically at 39°C for 24h with the addition of 10g of the ground (1-mm screen) diet described above as substrate and were performed in quadruplicate. Two vessels per treatment were randomly assigned. Anaerobic conditions were achieved by continuous infusion of N₂ at a rate of 20 ml/min. Maintaining the fermenters' temperature at 39°C and mixing of their contents were achieved by using the Takasugi Seisakusho-Culture fermenter base units (Takasugi Seisakusho Co. Ltd. Tokyo, Japan).

### <Supplement, inoculation and cultures of E. coli W3110 or E. coli nir-Ptac>

For the purpose of studying the effects of nitrate, nitrite, *E. coli* W3110, and *E. coli* nir-Ptac on *in vitro* ruminal methanogenesis by the ruminal bacteria , the incubation vessels were supplemented with nitrate (NaNO₃, 5 or 10mM) or nitrite (NaNO₂, 1 or 2mM) and inoculated with cultured *E. coli* W3110 or *E. coli* nir-Ptac. For the purpose of studying the effect of *E. coli* W3110 or *E. coli* nir-Ptac on *in vitro* ruminal nitrate and nitrite reduction by ruminal bacteria, cultured *E. coli* W3110 or *E. coli* nir-Ptac were inoculated into separate incubation vessels supplemented with nitrate (NaNO₃, 5 or 10mM) or nitrite (NaNO₂, 1 or 2mM). The cell amounts of *E. coli* W3110 or *E. coli* nir-Ptac inoculated into each incubation vessel were calculated as indicated in the below equation. The control culture was incubated without added nitrate and nitrite, and inoculated *E. coli* W3110 or *E. coli* nir-Ptac.

### E. coli nir-Ptac was constructed according to Example 1.

*E. coli* W3110 or *E. coli* nir-Ptac inoculum were prepared by culturing the cells in the Luria-Bertani (LB) broth agar at 37°C for 10hr. One-eight of *E. coli* W3110 or *E. coli* nir-Ptac cells on the agar plate were collected and put into flasks with 50ml of LB broth (10g of tryptone, 5g of yeast extract, 10g of NaCl per liter) and incubated for a further 12 hr at 37°C with constant shaking (150 rev. min⁻¹), respectively. Stationary phase cells were harvested with centrifugation (5000×g, 5 min, 4°C). Cell pellets of *E. coli* W3110 or *E. coli* nir-Ptac were suspended in 25ml of sterile buffer solution (pH 6.8) (McDaugall, 1948, Studies on ruminant saliva.1. The composition and output of sheep's saliva. Biochem. J. 43, 99-109) and re-suspended in 5ml of sterile buffer solution, respectively. The amount of the E. *coli* W3110 or E. *coli* nir-Ptac to be inoculated into each fermenter was calculated according to the equation: $E = ( A ) \times ( B ) / ( C ) \times ( D )$

Where E: the amount of the E. *coli* cells to be inoculated (ml).
A: OD₆₆₀ value of solution in fermenter assumed at 2.
B: the solution volume in fermenter (800ml).
C: OD₆₆₀ value of cultured E. *coli* W3110 or *E. coli* nir-Ptac cells diluted 1:200 with buffer solution (McDaugall, 1948 Studies on ruminant saliva.1. The composition and output of sheep's saliva. Biochem. J. 43, 99-109).
D: dilution rate (200)

According to the above calculation, E. *coli* W3110 or E. *coli* nir-Ptac cells inoculated into each fermenter ranged from 30.53 to 39.40ml (approximately 1 × 10⁹ cfu/ml).

### <Analytical procedures>

The above described materials were put into the *in vitro* rumen system to start the reaction. Rumen sample was collected at 0, 0.5, 1, 1.5, 2, 4, 6, 8, 10, 12, and 24h to immediately determine pH and cell growth (OD₆₆₀), and then each sample was stored at -20°C for later determination of ruminal nitrate, nitrite, ammonia nitrogen and volatile fatty acid. The value of pH in rumen fluid was measured using a pH meter (HM-21P, TOA Electronics Ltd., Tokyo, Japan). Optical density at 660nm (OD₆₆₀) was used as an index of cell growth of the bacterial population as well as of E. *coli* W3110 and E. *coli* nir-Ptac of the culture. Samples were diluted 1:200 with distilled water before measurement of OD₆₆₀ with spectrophotometer (Part No. 100-004, Serial No. 5667-15, Hitachi Ltd., Tokyo, Japan). Nitrate and nitrite concentration in culture supernatant was roughly estimated using NO₂- test paper (Merck: Merckoquant Nitrite Test 1. 10022.0001, 1.10007.0001) and NO₃⁻ test paper (Merck: Merckoquant Nitrate Test 1. 10020.0001), respectively. Then, the sample was diluted to fit the nitrate and nitrite concentration between 0 and 100µM which is the measurement range of the nitrate/nitrite Assay Kit-C (Colorimetric, Dojindo) and subject to the more accurate concentration measurement with the kit. The absorption coefficient was measured using the microplate reader SPECTRA MAX 190 (Molecular Devices). Ammonia nitrogen concentration was estimated as described previously (Sar et al., 2004 Manipulation of rumen methanogenesis by the combination of nitrate with β 1-4 galacto-oligosaccharides or nisin in sheep. Anim Feed Sci Technol (in press)). The concentrations of VFA were analyzed by gas-liquid chromatography (Shimadzu GC-14A, Kyoto, Japan) equipped with a flame-ionization detector and a capillary column (ULBON HR-52, 0.53 mm I.D. × 30 m 3.0 µm) by using 2-Ethyl-n-butyric acid as the internal standard. Values were calculated automatically using a Chromatopac data processing system (C-R 4A; Shimadzu). Continuous CH₄ and carbon dioxide (CO₂) production rate were automatically measured using an infrared gas analyzer (Takasugi Seisakusho Co. Ltd. Tokyo, Japan), and then those data were taken and pooled into the computer (Windows® XP Professional 1-2 CPU, IMB Corporation) from the analyzer through an interface at 1min intervals.

### <Statistical analysis>

*In vitro* continuous incubation in each treatment were performed for 2 days with two replicates per day (n=4). Cumulative CH₄ and CO₂ production were extrapolated by non-linear regression analyses of Bertalanffy model [(CH₄ (ml) or CO₂ (ml) = *a* + *b* (1-e^{*-ct*}) ³, where *a:* first CH₄ or CO₂ production, *b*: second CH₄ or CO₂ production, c: constant CH₄ and CO₂ production rate, t: time (min)) from the time course of CH₄ or CO₂ production for 24h incubation. Means of results from treatments were analyzed by one-way analysis of variance (ANOVA) using the General Linear Models Procedures of the Statistical Analysis Systems Institute (SAS, 1994). Treatment means were statistically compared with Duncan's multiple-range test. Differences with P < 0.05 were considered significant.

### <Result>

The result of Example 2 is shown in Table 1.

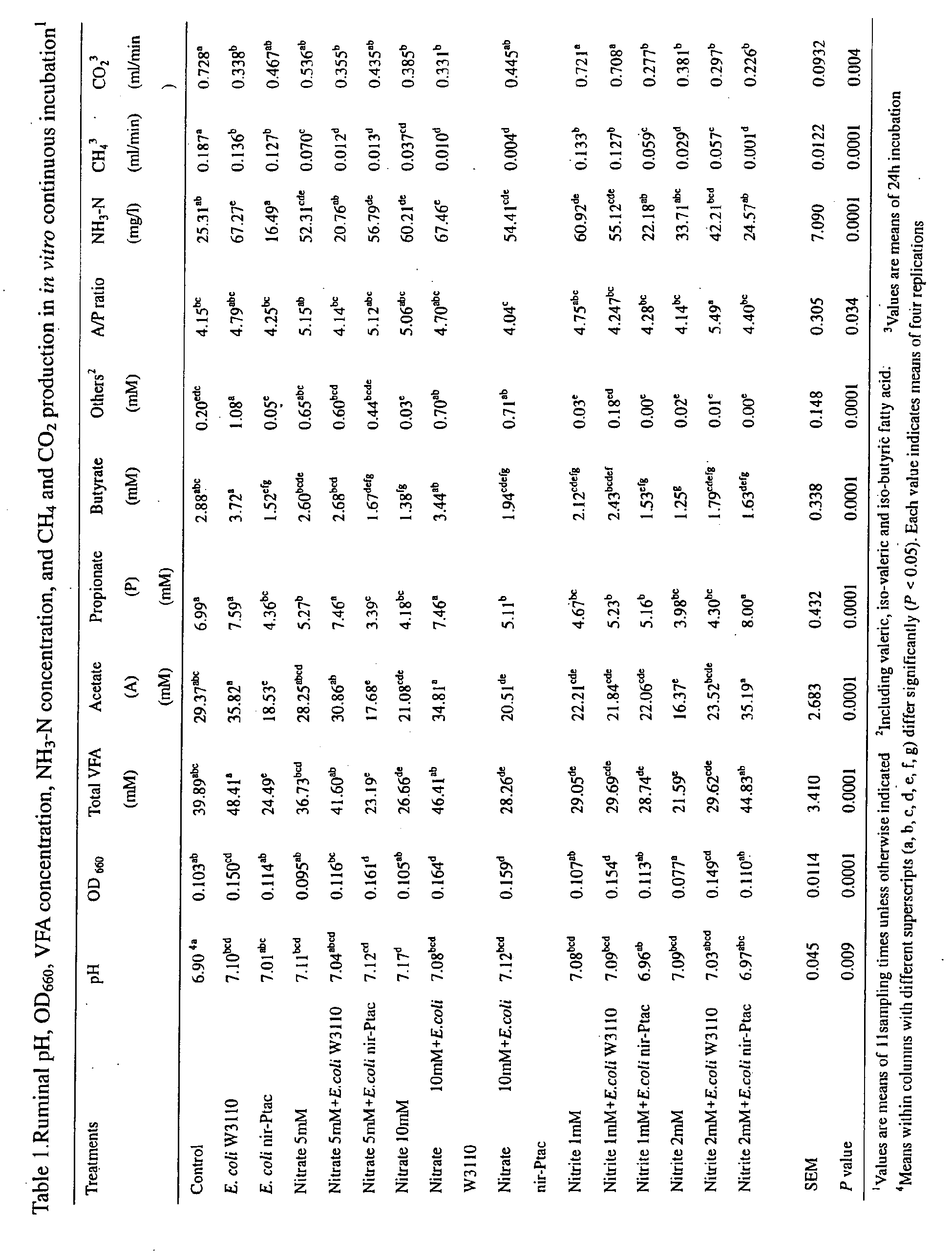

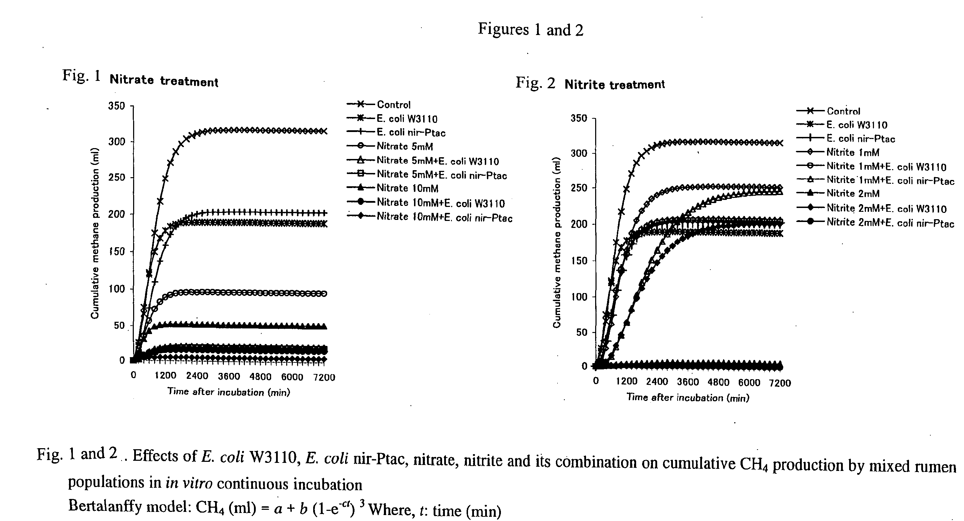

Figure 1 and Figure 2 show profiles of *in vitro* cumulative CH₄ production in cultures of mixed ruminal bacteria treated with nitrate and nitrite, respectively. Cumulative CH₄ production was reduced in all treatments compared to control incubation. When E. *coli* W3110 was inoculated to 5 or 10mM-nitrate, reduced cumulative CH₄ production was observed compared to 5 or 10mM-nitrate without inoculation of the strain. Cumulative CH₄ production was drastically decreased by inoculation of E. *coli* nir-Ptac to 5 or 10mM-nitrate and 1 or 2mM-nitrite compared to 5 or 10mM-nitrate and 1 or 2mM-nitrite without inoculation of the strain, respectively.

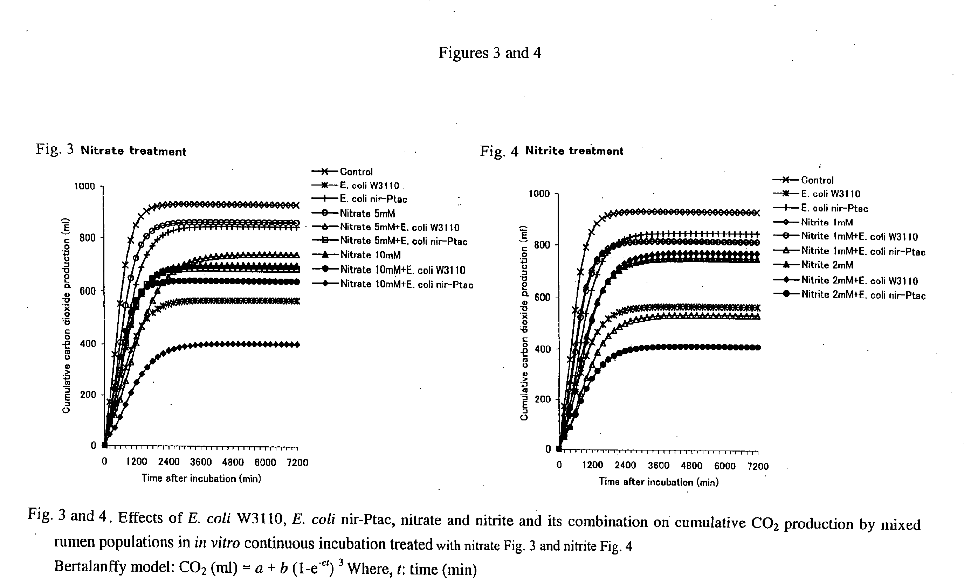

Figure 3 and Figure 4 show profiles of *in vitro* cumulative CO₂ production in cultures of mixed ruminal bacteria treated with nitrate and nitrite, respectively. Reduced cumulative CO₂ production in all treatments was observed compared to control incubation. In treatments of nitrate, the lowest cumulative CO₂ production was obtained in 10mM-nitrate plus E. *coli* nir-Ptac. In treatments of nitrite, cumulative CO₂ production was drastically reduced to greatest extents in 2mM-nitrite plus E. *coli* nir-Ptac.

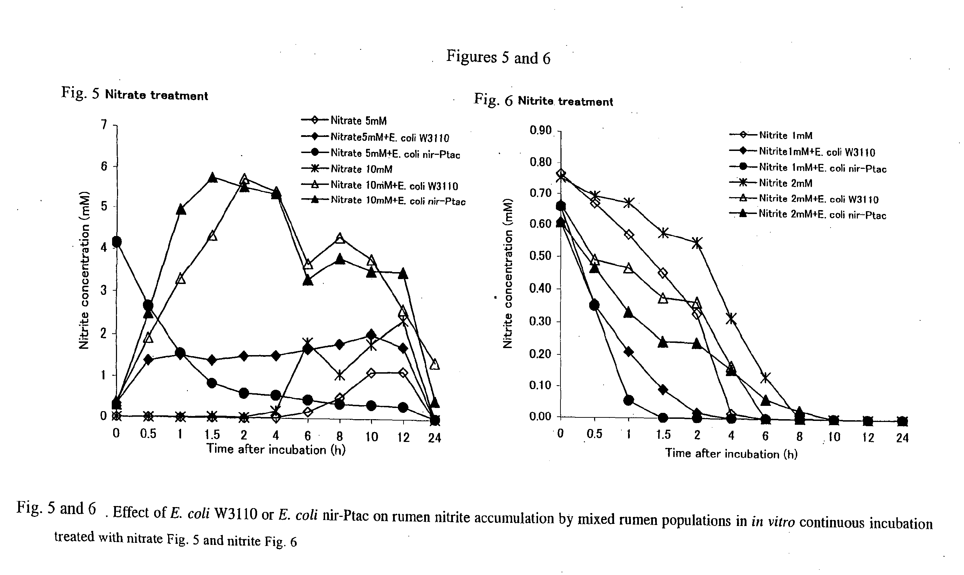

Figure 5 and Figure 6 show profiles of ruminal nitrite accumulation in *in vitro* continuous incubation of mixed ruminal bacteria treated with nitrate and nitrite, respectively. In the treatment of nitrate, an inoculation of E. *coli* W3110 and E. *coli* nir-Ptac to mixed rumen population treated with 5 or 10mM-nitrate resulted in an (P > 0.05) increase in ruminal nitrite concentration compared to 5 or 10mM-nitrate without inoculation of those strains, respectively. In the treatment of nitrite, ruminal nitrite concentration in 1mM-nitrite plus E. *coli* W3110 decreased (P < 0.01) compared to 1 mM-nitrite without inoculation of the strain, the (*P* < 0.01) greatest values corresponding to 1mM-nitrite plus E. *coli* nir-Ptac. Compared to 2mM-nitrite, the ruminal nitrite concentration was decreased (*P* < 0.01) with 2mM-nitrite plus E. *coli* W3110, the greatest (P < 0.01) values being found in 2mM-nitrite plus E. *coli* nir-Ptac.

The above examples show that wild-type E. *coli* W3110 and E. *coli* nir-Ptac reduce the amount of nitrite in rumen, repress negative effect of nitrate and nitrite, and reduce *in vitro* ruminal methane production.
ni5 : tca gcc gtc acc gtc agc ata aca c (SEQ ID NO: 1)
nic4.1 : ccg aca ggc gtg caa tgc gcg cag c (SEQ ID NO: 2)
nirex5 : aaa aga att cga ggc aaa aat gag caa agt (SEQ ID NO: 3)
nirex3 : ccc caa gct tca tgc aaa aag ggg agg cat (SEQ ID NO: 4)
pKK-c200 : cgg ggt acc ttc tgg cgt cag gca gcc at (SEQ ID NO: 5)
NI'-c2035 : aca tgc atg ccg tct acg ccc agc agt ttc (SEQ ID NO: 6)
NI'-1 : cgg aat tcg tat gaa ggg cgt cag cgc g (SEQ ID NO: 7)
NI'-c925 : cgg ggt acc ttc tta agt cac gga att gt (SEQ ID NO: 8)
*Escherichia coli Nir*BD operon *Nir*B gene (SEQ ID NO: 9)
*Escherichia coli Nir*B protein (SEQ ID NO: 10)
*Escherichia coli Nir*D gene (SEQ ID NO: 11)
*Escherichia coli Nir*D protein (SEQ ID NO: 12)
*Escherichia coli Nrf*ABCDEFG operon (SEQ ID NO: 13)
*Escherichia coli Nrf*A protein (SEQ ID NO: 14)
*Escherichia coli Nrf*B protein (SEQ ID NO: 15)
*Escherichia coli Nrf*C protein (SEQ ID NO: 16)
*Escherichia coli Nrf*D protein (SEQ ID NO: 17)
*Escherichia coli Nrf*E protein (SEQ ID NO: 18)
*Escherichia coli Nrf*F protein (SEQ ID NO: 19)
*Escherichia coli Nrf*G protein (SEQ ID NO: 20)
*Corynebacterium glutamicum Nir*A gene (SEQ ID NO: 21)
*Corynebacterium glutamicum NirA* protein (SEQ ID NO: 22

## Claims

1. A feed composition for ruminants comprising a microbe having nitrite reductase activity, wherein said microbe has been modified so that intracellular nitrite reductase activity is enhanced.

2. The feed composition of claim 1, wherein said microbe is selected from the group consisting of intestinal bacteria, coryneform bacteria, *Bacillus bacterium,* bacteria of the genus *Methylophilus, Actinomyces,* ruminal bacteria, and a combination thereof.

3. The feed composition of claim 1, wherein said microbe is Escherichia bacterium.

4. The feed composition of claim 1, wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20, 22, and combination thereof, except that 1 to 30 amino acids have been substituted, deleted, inserted, or added, and wherein said protein has nitrite reductase activity.

5. The feed composition of claim 1, wherein said microbe is **characterized by** enhanced expression of a gene coding for nitrite reductase.

6. The feed composition of claim 5, wherein said gene coding for nitrite reductase is derived from a bacterium of the genus *Escherichia,* a coryneform bacterium, or a bacterium of the genus *Bacillus.*

7. The feed composition of claim 6, wherein said gene coding for said nitrite reductase comprises a nucleotide sequence selected from the group consisting of:
(A)a nucleotide sequence selected from the group consisting of SEQ ID NO: 9, 13, and 21; and
(B)a nucleotide sequence which is able to hybrid under stringent conditions with the nucleotide sequence selected from the group consisting of SEQ ID NO: 9, 13, and 21, or with a probe prepared from one of said nucleotide sequences, wherein said gene codes for a protein having nitrite reductase activity.

8. The use of a microbe having nitrite reductase activity, wherein said microbe has been modified so that intracellular nitrite reductase activity is enhanced for the preparation of a feed composition for improving the growth of a ruminant.

9. The use of claim 8, wherein said microbe has been modified so that intracellular nitrite reductase activity is enhanced.

10. The use of claim 8,wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20., 22, and combination thereof, except that 1 to 30 amino acids have been substituted, deleted, inserted, or added, and wherein said protein has nitrite reductase activity.

11. The use of claim 8, wherein said microbe is **characterized by** enhanced expression of a gene coding for nitrite reductase.

12. The use of claim 11, wherein said gene coding for nitrite reductase is derived from a bacterium of the genus *Escherichia,* a coryneform bacterium, or a bacterium of the genus *Bacillus.*

13. The use of Claim 12, wherein said gene coding for said nitrite reductase comprises a nucleotide sequence selected from the group consisting of:
(A) a nucleotide sequence selected from the group consisting of SEQ ID NO: 9, 13, and 21; and
(B) a nucleotide sequence which is able to hybrid under stringent conditions with the nucleotide sequence selected from the group consisting of SEQ ID NO: 9, 13, and 21, or with a probe prepared from one of said nucleotide sequences, wherein said DNA codes for a protein having nitrite reductase activity.

14. The use of a microbe having nitrite reductase activity, wherein said microbe has been modified so that intracellular nitrite reductase activity is enhanced for the preparation of a feed composition for preventing nitrite intoxication in a ruminant.

15. The use of claim 14, wherein said microbe has been modified so that intracellular nitrite reductase activity is enhanced.

16. The use of claim 15, wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20, 22, and combination thereof, except that 1 to 30 amino acids have been substituted, deleted, inserted, or added, and wherein said protein has nitrite reductase activity.

17. The use of claim 14 wherein said microbe is **characterized by** enhanced expression of a gene coding for nitrite reductase.

18. A feed composition for ruminants comprising a microbe having nitrite reductase activity, wherein said microbe is selected from the group consisting of intestinal bacteria, coryneform bacteria, *Bacillus subtilis,* bacteria of the genus *Methylophilus, Actinomyces,* ruminal bacteria, and a combination thereof.

19. The feed composition of claim 18 wherein said nitrite reductase comprises a protein selected from the group consisting of:
(A) a protein selected from the group consisting of SEQ ID NO: 10, 12, 14 to 20, 22, and combination thereof; and
(B) a protein selected from the group consisting of SEQ ID NO: 10, 12, 14 to 20, 22, and combination thereof, except that 1 to 30 amino acids have been substituted, deleted, inserted, or added, and wherein said protein has nitrite reductase activity.

20. The use of a microbe having nitrite reductase activity, wherein said microbe is selected from the group consisting of intestinal bacteria, coryneform bacteria, Bacillus subtilis, bacteria of the genus Methylophilus, Actinomyces, ruminal bacteria, and a combination thereof for the preparation of a feed composition for improving the growth of a ruminant.

21. The use of a microbe having nitrite reductase activity, wherein said microbe is selected from the group consisting of intestinal bacteria, coryneform bacteria, Bacillus subtilis, bacteria of the genus Methylophilus, Actinomyces, ruminal bacteria, and a combination thereof for the preparation of a feed composition for preventing nitrite intoxication in a ruminant.


SEQUENCE LISTING

<110>  Ajinomoto Co., Inc
<120>  Methane Production Inhibitor and Feed Composition for Ruminants

<130>  C-439/YM

<150>  JP2004-245705
<151>  2004-08-25

<160>  22

<170>  PatentIn version 3.1

<210>  1
<211>  25
<212>  DNA
<213>  Artificial sequence

<220>
<223>  primer

<400>  1
tcagccgtca ccgtcagcat aacac                                          25

<210>  2
<211>  25
<212>  DNA
<213>  Artificial sequence

<220>
<223>  primer

<400>  2
ccgacaggcg tgcaatgcgc gcagc                                          25

<210>  3
<211>  30
<212>  DNA
<213>  Artificial sequence

<220>
<223>  primer

<400>  3
aaaagaattc gaggcaaaaa tgagcaaagt                                     30

<210>  4
<211>  30
<212>  DNA
<213>  Artificial sequence

<220>
<223>  primer

<400>  4
ccccaagctt catgcaaaaa ggggaggcat                                     30

<210>  5
<211>  29
<212>  DNA
<213>  Artificial sequence

<220>
<223>  primer

<400>  5
cggggtacct tctggcgtca ggcagccat                                      29

<210>  6

<211>  30
<212>  DNA
<213>  Artificial sequence

<220>
<223>  primer

<400>  6
acatgcatgc cgtctacgcc cagcagtttc                                        30


<210>  7
<211>  28
<212>  DNA
<213>  Artificial sequence

<220>
<223>  primer

<400>  7
cggaattcgt atgaagggcg tcagcgcg                                          28


<210>  8
<211>  29
<212>  DNA
<213>  Artificial sequence

<220>
<223>  primer

<400>  8
cggggtacct tcttaagtca cggaattgt                                         29


<210>  9
<211>  2867
<212>  DNA
<213>  Escherichia coli

<220>
<221>  CDS
<222>  (1)..(2541)
<223>

<400>  9
atg agc aaa gtc aga ctc gca att atc ggt aac ggt atg gtc ggc cat      48
Met Ser Lys Val Arg Leu Ala Ile Ile Gly Asn Gly Met Val Gly His
1               5                   10                  15

cgc ttt atc gaa gat ctt ctt gat aaa tct gat gcg gcc aac ttt gat      96
Arg Phe Ile Glu Asp Leu Leu Asp Lys Ser Asp Ala Ala Asn Phe Asp
            20                  25                  30

att acc gtt ttc tgt gaa gaa ccg cgc atc gct tat gac cgc gta cac      144
Ile Thr Val Phe Cys Glu Glu Pro Arg Ile Ala Tyr Asp Arg Val His
        35                  40                  45

ctc tcg tct tac ttc tct cac cac acc gcc gaa gag ctg tcg ctg gtg      192
Leu Ser Ser Tyr Phe Ser His His Thr Ala Glu Glu Leu Ser Leu Val
    50                  55                  60

cgc gaa ggc ttc tac gag aaa cac ggc atc aaa gtt ctg gtc ggc gaa      240
Arg Glu Gly Phe Tyr Glu Lys His Gly Ile Lys Val Leu Val Gly Glu
65                  70                  75                  80

cgc gct atc acc atc aac cgt cag gag aag gtg att cac tcc agc gcc      288
Arg Ala Ile Thr Ile Asn Arg Gln Glu Lys Val Ile His Ser Ser Ala
                85                  90                  95

gga cgt acc gtt ttt tat gac aag ctg atc atg gca acc ggt tcc tac      336
Gly Arg Thr Val Phe Tyr Asp Lys Leu Ile Met Ala Thr Gly Ser Tyr
            100                 105                 110

ccg tgg atc ccg cca atc aaa ggt tct gat act cag gac tgc ttt gtc      384

Pro Trp Ile Pro Pro Ile Lys Gly Ser Asp Thr Gln Asp Cys Phe Val
        115                 120                 125

tat cgc act att gaa gac ctc aac gcc att gaa tcc tgc gcc cgt cgc 432
Tyr Arg Thr Ile Glu Asp Leu Asn Ala Ile Glu Ser Cys Ala Arg Arg
    130                 135                 140

agc aaa cgc ggt gcc gtt gtt ggt ggc ggc ctg tta ggt ctg gaa gcc 480
Ser Lys Arg Gly Ala Val Val Gly Gly Gly Leu Leu Gly Leu Glu Ala
145                 150                 155                 160

gca ggc gcg ctg aaa aac tta ggt att gaa acc cac gtt atc gaa ttt 528
Ala Gly Ala Leu Lys Asn Leu Gly Ile Glu Thr His Val Ile Glu Phe
                165                 170                 175

gcc cct atg ctg atg gca gaa cag ctt gat cag atg ggc ggc gag cag 576
Ala Pro Met Leu Met Ala Glu Gln Leu Asp Gln Met Gly Gly Glu Gln
            180                 185                 190

ctg cgt cgc aaa atc gaa agt atg ggc gtg cgc gtt cac acc agc aaa 624
Leu Arg Arg Lys Ile Glu Ser Met Gly Val Arg Val His Thr Ser Lys
        195                 200                 205

aac acc ctt gag att gtg cag gaa ggt gtt gaa gcg cgt aaa acc atg 672
Asn Thr Leu Glu Ile Val Gln Glu Gly Val Glu Ala Arg Lys Thr Met
    210                 215                 220

cgt ttt gcc gac ggc agc gaa ctg gaa gtc gac ttt atc gtc ttc tct 720
Arg Phe Ala Asp Gly Ser Glu Leu Glu Val Asp Phe Ile Val Phe Ser
225                 230                 235                 240

acc ggt atc cgt ccg cgc gat aag ctg gca acc cag tgt ggt ctg gac 768
Thr Gly Ile Arg Pro Arg Asp Lys Leu Ala Thr Gln Cys Gly Leu Asp
                245                 250                 255

gtt gct ccg cgt ggg ggt att gtc att aat gat tcc tgc cag act tcc 816
Val Ala Pro Arg Gly Gly Ile Val Ile Asn Asp Ser Cys Gln Thr Ser
            260                 265                 270

gat ccg gat atc tac gcc atc ggt gaa tgc gca agc tgg aac aac cgt 864
Asp Pro Asp Ile Tyr Ala Ile Gly Glu Cys Ala Ser Trp Asn Asn Arg
        275                 280                 285

gta ttt ggt ctg gta gca cct ggc tac aaa atg gcg cag gtc gcc gtt 912
Val Phe Gly Leu Val Ala Pro Gly Tyr Lys Met Ala Gln Val Ala Val
    290                 295                 300

gac cat att ctc ggt agc gaa aac gcc ttt gaa ggt gct gac ctt agc 960
Asp His Ile Leu Gly Ser Glu Asn Ala Phe Glu Gly Ala Asp Leu Ser
305                 310                 315                 320

gcc aag ctg aaa ctg ctg ggc gta gac gta ggc ggt att ggt gat gcg 1008
Ala Lys Leu Lys Leu Leu Gly Val Asp Val Gly Gly Ile Gly Asp Ala
                325                 330                 335

cac ggt cgc acg cct ggc gca cgt agc tac gtt tac ctc gac gaa agt 1056
His Gly Arg Thr Pro Gly Ala Arg Ser Tyr Val Tyr Leu Asp Glu Ser
            340                 345                 350

aaa gag atc tac aaa cgc ctg att gtc agc gaa gac aac aaa acc ctg 1104
Lys Glu Ile Tyr Lys Arg Leu Ile Val Ser Glu Asp Asn Lys Thr Leu
        355                 360                 365

ctc ggt gcg gta ctg gtg ggc gat acc agc gac tac ggt aac ctg ctg 1152
Leu Gly Ala Val Leu Val Gly Asp Thr Ser Asp Tyr Gly Asn Leu Leu
    370                 375                 380

caa ctg gtg ctg aac gct atc gaa ctg ccg gaa aac ccg gat tcc ctg 1200
Gln Leu Val Leu Asn Ala Ile Glu Leu Pro Glu Asn Pro Asp Ser Leu
385                 390                 395                 400

atc ctg cca gca cac tcg ggt agc ggc aag ccg tct atc ggt gtt gat 1248
Ile Leu Pro Ala His Ser Gly Ser Gly Lys Pro Ser Ile Gly Val Asp
                405                 410                 415

aaa ctg ccg gac agc gcg caa atc tgc tcc tgc ttc gac gtc acc aaa 1296
Lys Leu Pro Asp Ser Ala Gln Ile Cys Ser Cys Phe Asp Val Thr Lys
            420                 425                 430

ggt gat ctg att gct gcc atc aac aaa ggc tgc cac aca gtt gcg gcg 1344
Gly Asp Leu Ile Ala Ala Ile Asn Lys Gly Cys His Thr Val Ala Ala
        435                 440                 445

ctg aaa gct gaa acc aaa gcg ggt act ggc tgc ggt ggc tgt atc ccg 1392
Leu Lys Ala Glu Thr Lys Ala Gly Thr Gly Cys Gly Gly Cys Ile Pro
    450                 455                 460

ctg gtc act cag gta ctg aac gcg gaa ctg gcg aaa cag ggc atc gaa 1440
Leu Val Thr Gln Val Leu Asn Ala Glu Leu Ala Lys Gln Gly Ile Glu
465                 470                 475                 480

gtt aac aac aac ctg tgc gaa cac ttt gct tat tcg cgt cag gaa ctg 1488
Val Asn Asn Asn Leu Cys Glu His Phe Ala Tyr Ser Arg Gln Glu Leu
                485                 490                 495

ttc cat ttg atc cgc gtt gaa ggc att aaa acc ttc gaa gaa ctg ctg 1536
Phe His Leu Ile Arg Val Glu Gly Ile Lys Thr Phe Glu Glu Leu Leu
            500                 505                 510

gcg aaa cac ggc aaa ggc tac ggt tgt gaa gtt tgt aaa cca acc gtc 1584
Ala Lys His Gly Lys Gly Tyr Gly Cys Glu Val Cys Lys Pro Thr Val
        515                 520                 525

ggt tcg ctg ctg gcc tcc tgc tgg aac gaa tac att ctg aag ccg gaa 1632
Gly Ser Leu Leu Ala Ser Cys Trp Asn Glu Tyr Ile Leu Lys Pro Glu
    530                 535                 540

cat act ccg ctg cag gat tct aac gac aac ttc ctc gct aac atc cag 1680
His Thr Pro Leu Gln Asp Ser Asn Asp Asn Phe Leu Ala Asn Ile Gln
545                 550                 555                 560

aaa gac ggc acc tac tcg gtg atc ccg cgt tct ccg ggc ggt gaa atc 1728
Lys Asp Gly Thr Tyr Ser Val Ile Pro Arg Ser Pro Gly Gly Glu Ile
                565                 570                 575

acc ccg gaa ggg ctg atg gcg gta ggt cgt atc gcg cgt gaa ttt aat 1776
Thr Pro Glu Gly Leu Met Ala Val Gly Arg Ile Ala Arg Glu Phe Asn
            580                 585                 590

ctc tac acc aag atc act ggc tcc cag cgt ctg gcg atg ttt ggc gca 1824
Leu Tyr Thr Lys Ile Thr Gly Ser Gln Arg Leu Ala Met Phe Gly Ala
        595                 600                 605

cag aaa gac gat ctg ccg gag atc tgg cgt cag ctg att gaa gcc ggc 1872
Gln Lys Asp Asp Leu Pro Glu Ile Trp Arg Gln Leu Ile Glu Ala Gly
    610                 615                 620

ttc gaa acc ggt cat gcc tat gcg aaa gca ctg cgt atg gcg aaa acc 1920
Phe Glu Thr Gly His Ala Tyr Ala Lys Ala Leu Arg Met Ala Lys Thr
625                 630                 635                 640

tgc gtg ggt agc acc tgg tgc cgc tac ggc gtt ggc gac agc gtc ggc 1968
Cys Val Gly Ser Thr Trp Cys Arg Tyr Gly Val Gly Asp Ser Val Gly
                645                 650                 655

ctc ggc gtg gaa ctg gaa aac cgc tac aaa ggc atc cgt acg ccg cac 2016
Leu Gly Val Glu Leu Glu Asn Arg Tyr Lys Gly Ile Arg Thr Pro His
            660                 665                 670

aaa atg aag ttc ggt gtc tcc ggc tgt acc cgt gaa tgt tca gaa gct 2064
Lys Met Lys Phe Gly Val Ser Gly Cys Thr Arg Glu Cys Ser Glu Ala
        675                 680                 685

cag ggt aaa gac gtg ggt att atc gcc act gaa aaa ggc tgg aac ctg 2112
Gln Gly Lys Asp Val Gly Ile Ile Ala Thr Glu Lys Gly Trp Asn Leu
    690                 695                 700

tat gtt tgc ggt aac ggc ggc atg aaa ccg cgt cat gcg gat ctg ctg 2160
Tyr Val Cys Gly Asn Gly Gly Met Lys Pro Arg His Ala Asp Leu Leu
705                 710                 715                 720

gcg gcg gat atc gat cgc gaa acg ctg atc aaa tat ctc gac cgc ttc     2208
Ala Ala Asp Ile Asp Arg Glu Thr Leu Ile Lys Tyr Leu Asp Arg Phe
                725                 730                 735

atg atg ttc tac atc cgt act gcc gac aaa ctg acg cgt acc gca ccg     2256
Met Met Phe Tyr Ile Arg Thr Ala Asp Lys Leu Thr Arg Thr Ala Pro
            740                 745                 750

tgg tta gaa aac ctc gaa ggc ggc atc gat tac ctg aaa gca gtg atc     2304
Trp Leu Glu Asn Leu Glu Gly Gly Ile Asp Tyr Leu Lys Ala Val Ile
        755                 760                 765

att gac gac aaa ctg ggg ctg aac gca cat ctg gaa gaa gag atg gcg     2352
Ile Asp Asp Lys Leu Gly Leu Asn Ala His Leu Glu Glu Glu Met Ala
    770                 775                 780

cgc ctg cgt gaa gcg gta ctg tgt gag tgg act gaa acg gtc aat acg     2400
Arg Leu Arg Glu Ala Val Leu Cys Glu Trp Thr Glu Thr Val Asn Thr
785                 790                 795                 800

ccg tct gcg cag act cgc ttc aaa cac ttc atc aac agc gac aag cgt     2448
Pro Ser Ala Gln Thr Arg Phe Lys His Phe Ile Asn Ser Asp Lys Arg
                805                 810                 815

gac ccg aac gtg cag atg gtg cca gag cgc gaa cag cac cgt ccg gca     2496
Asp Pro Asn Val Gln Met Val Pro Glu Arg Glu Gln His Arg Pro Ala
            820                 825                 830

acg ccg tat gaa cgt atc cca gta act ctg gtg gag gac aac gca         2541
Thr Pro Tyr Glu Arg Ile Pro Val Thr Leu Val Glu Asp Asn Ala
        835                 840                 845

tgagccagtg gaaagacatc tgcaaaatcg atgacatcct gcctgaaacc ggcgtctgcg   2601

cgctgttagg tgacgagcag gtcgcgattt tccgcccgta tcacagcgat caggtgtttg   2661

cgatcagcaa catcgacccg ttcttcgagt ccagcgtgct gtcacgcgga ctgattgcgg   2721

aacaccaggg cgagctgtgg gtcgccagcc cgctgaaaaa acagcgtttt cgcttaagcg   2781

acggcttgtg catggaagac gaacagtttt ccgtcaaaca ttacgaagcg cgagtgaaag   2841

acggcgtggt gcagctgcgc ggttaa                                        2867

<210>  10
<211>  847
<212>  PRT
<213>  Escherichia coli

<400>  10

Met Ser Lys Val Arg Leu Ala Ile Ile Gly Asn Gly Met Val Gly His
1               5                   10                  15

Arg Phe Ile Glu Asp Leu Leu Asp Lys Ser Asp Ala Ala Asn Phe Asp
            20                  25                  30

Ile Thr Val Phe Cys Glu Glu Pro Arg Ile Ala Tyr Asp Arg Val His
        35                  40                  45

Leu Ser Ser Tyr Phe Ser His His Thr Ala Glu Glu Leu Ser Leu Val
    50                  55                  60

Arg Glu Gly Phe Tyr Glu Lys His Gly Ile Lys Val Leu Val Gly Glu
65                  70                  75                  80

Arg Ala Ile Thr Ile Asn Arg Gln Glu Lys Val Ile His Ser Ser Ala
                85                  90                  95

Gly Arg Thr Val Phe Tyr Asp Lys Leu Ile Met Ala Thr Gly Ser Tyr
            100                 105                 110

Pro Trp Ile Pro Pro Ile Lys Gly Ser Asp Thr Gln Asp Cys Phe Val
        115                 120                 125

Tyr Arg Thr Ile Glu Asp Leu Asn Ala Ile Glu Ser Cys Ala Arg Arg
    130                 135                 140

Ser Lys Arg Gly Ala Val Val Gly Gly Gly Leu Leu Gly Leu Glu Ala
145                 150                 155                 160

Ala Gly Ala Leu Lys Asn Leu Gly Ile Glu Thr His Val Ile Glu Phe
                165                 170                 175

Ala Pro Met Leu Met Ala Glu Gln Leu Asp Gln Met Gly Gly Glu Gln
            180                 185                 190

Leu Arg Arg Lys Ile Glu Ser Met Gly Val Arg Val His Thr Ser Lys
        195                 200                 205

Asn Thr Leu Glu Ile Val Gln Glu Gly Val Glu Ala Arg Lys Thr Met
    210                 215                 220

Arg Phe Ala Asp Gly Ser Glu Leu Glu Val Asp Phe Ile Val Phe Ser
225                 230                 235                 240

Thr Gly Ile Arg Pro Arg Asp Lys Leu Ala Thr Gln Cys Gly Leu Asp
                245                 250                 255

Val Ala Pro Arg Gly Gly Ile Val Ile Asn Asp Ser Cys Gln Thr Ser
            260                 265                 270

Asp Pro Asp Ile Tyr Ala Ile Gly Glu Cys Ala Ser Trp Asn Asn Arg
        275                 280                 285

Val Phe Gly Leu Val Ala Pro Gly Tyr Lys Met Ala Gln Val Ala Val
    290                 295                 300

Asp His Ile Leu Gly Ser Glu Asn Ala Phe Glu Gly Ala Asp Leu Ser
305                 310                 315                 320

Ala Lys Leu Lys Leu Leu Gly Val Asp Val Gly Gly Ile Gly Asp Ala
                325                 330                 335

His Gly Arg Thr Pro Gly Ala Arg Ser Tyr Val Tyr Leu Asp Glu Ser
            340                 345                 350

Lys Glu Ile Tyr Lys Arg Leu Ile Val Ser Glu Asp Asn Lys Thr Leu
        355                 360                 365

Leu Gly Ala Val Leu Val Gly Asp Thr Ser Asp Tyr Gly Asn Leu Leu
    370                 375                 380

Gln Leu Val Leu Asn Ala Ile Glu Leu Pro Glu Asn Pro Asp Ser Leu

385                 390                 395                 400

Ile Leu Pro Ala His Ser Gly Ser Gly Lys Pro Ser Ile Gly Val Asp
                405                 410                 415

Lys Leu Pro Asp Ser Ala Gln Ile Cys Ser Cys Phe Asp Val Thr Lys
            420                 425                 430

Gly Asp Leu Ile Ala Ala Ile Asn Lys Gly Cys His Thr Val Ala Ala
        435                 440                 445

Leu Lys Ala Glu Thr Lys Ala Gly Thr Gly Cys Gly Gly Cys Ile Pro
    450                 455                 460

Leu Val Thr Gln Val Leu Asn Ala Glu Leu Ala Lys Gln Gly Ile Glu
465                 470                 475                 480

Val Asn Asn Asn Leu Cys Glu His Phe Ala Tyr Ser Arg Gln Glu Leu
                485                 490                 495

Phe His Leu Ile Arg Val Glu Gly Ile Lys Thr Phe Glu Glu Leu Leu
            500                 505                 510

Ala Lys His Gly Lys Gly Tyr Gly Cys Glu Val Cys Lys Pro Thr Val
        515                 520                 525

Gly Ser Leu Leu Ala Ser Cys Trp Asn Glu Tyr Ile Leu Lys Pro Glu
    530                 535                 540

His Thr Pro Leu Gln Asp Ser Asn Asp Asn Phe Leu Ala Asn Ile Gln
545                 550                 555                 560

Lys Asp Gly Thr Tyr Ser Val Ile Pro Arg Ser Pro Gly Gly Glu Ile
                565                 570                 575

Thr Pro Glu Gly Leu Met Ala Val Gly Arg Ile Ala Arg Glu Phe Asn
            580                 585                 590

Leu Tyr Thr Lys Ile Thr Gly Ser Gln Arg Leu Ala Met Phe Gly Ala
        595                 600                 605

Gln Lys Asp Asp Leu Pro Glu Ile Trp Arg Gln Leu Ile Glu Ala Gly
    610                 615                 620

Phe Glu Thr Gly His Ala Tyr Ala Lys Ala Leu Arg Met Ala Lys Thr
625                 630                 635                 640

Cys Val Gly Ser Thr Trp Cys Arg Tyr Gly Val Gly Asp Ser Val Gly
                645                 650                 655

Leu Gly Val Glu Leu Glu Asn Arg Tyr Lys Gly Ile Arg Thr Pro His
            660                 665                 670

Lys Met Lys Phe Gly Val Ser Gly Cys Thr Arg Glu Cys Ser Glu Ala
        675                 680                 685

Gln Gly Lys Asp Val Gly Ile Ile Ala Thr Glu Lys Gly Trp Asn Leu
    690                 695                 700

Tyr Val Cys Gly Asn Gly Gly Met Lys Pro Arg His Ala Asp Leu Leu
705                 710                 715                 720

Ala Ala Asp Ile Asp Arg Glu Thr Leu Ile Lys Tyr Leu Asp Arg Phe
                725                 730                 735

Met Met Phe Tyr Ile Arg Thr Ala Asp Lys Leu Thr Arg Thr Ala Pro
            740                 745                 750

Trp Leu Glu Asn Leu Glu Gly Gly Ile Asp Tyr Leu Lys Ala Val Ile
        755                 760                 765

Ile Asp Asp Lys Leu Gly Leu Asn Ala His Leu Glu Glu Glu Met Ala
    770                 775                 780

Arg Leu Arg Glu Ala Val Leu Cys Glu Trp Thr Glu Thr Val Asn Thr
785                 790                 795                 800

Pro Ser Ala Gln Thr Arg Phe Lys His Phe Ile Asn Ser Asp Lys Arg
                805                 810                 815

Asp Pro Asn Val Gln Met Val Pro Glu Arg Glu Gln His Arg Pro Ala
            820                 825                 830

Thr Pro Tyr Glu Arg Ile Pro Val Thr Leu Val Glu Asp Asn Ala
        835                 840                 845

<210> 11
<211> 327
<212> DNA
<213> Escherichia coli

<220>
<221> CDS
<222> (1)..(324)
<223>

<400> 11
atg agc cag tgg aaa gac atc tgc aaa atc gat gac atc ctg cct gaa 48
Met Ser Gln Trp Lys Asp Ile Cys Lys Ile Asp Asp Ile Leu Pro Glu
1               5                   10                  15

acc ggc gtc tgc gcg ctg tta ggt gac gag cag gtc gcg att ttc cgc 96
Thr Gly Val Cys Ala Leu Leu Gly Asp Glu Gln Val Ala Ile Phe Arg
            20                  25                  30

ccg tat cac agc gat cag gtg ttt gcg atc agc aac atc gac ccg ttc 144
Pro Tyr His Ser Asp Gln Val Phe Ala Ile Ser Asn Ile Asp Pro Phe
        35                  40                  45

ttc gag tcc agc gtg ctg tca cgc gga ctg att gcg gaa cac cag ggc 192
Phe Glu Ser Ser Val Leu Ser Arg Gly Leu Ile Ala Glu His Gln Gly
    50                  55                  60

gag ctg tgg gtc gcc agc ccg ctg aaa aaa cag cgt ttt cgc tta agc 240
Glu Leu Trp Val Ala Ser Pro Leu Lys Lys Gln Arg Phe Arg Leu Ser
65                  70                  75                  80

gac ggc ttg tgc atg gaa gac gaa cag ttt tcc gtc aaa cat tac gaa 288
Asp Gly Leu Cys Met Glu Asp Glu Gln Phe Ser Val Lys His Tyr Glu
                85                  90                  95

gcg cga gtg aaa gac ggc gtg gtg cag ctg cgc ggt taa                327
Ala Arg Val Lys Asp Gly Val Val Gln Leu Arg Gly
            100                 105

<210>  12
<211>  108
<212>  PRT
<213>  Escherichia coli

<400>  12

Met Ser Gln Trp Lys Asp Ile Cys Lys Ile Asp Asp Ile Leu Pro Glu
1               5                   10                  15

Thr Gly Val Cys Ala Leu Leu Gly Asp Glu Gln Val Ala Ile Phe Arg
            20                  25                  30

Pro Tyr His Ser Asp Gln Val Phe Ala Ile Ser Asn Ile Asp Pro Phe
        35                  40                  45

Phe Glu Ser Ser Val Leu Ser Arg Gly Leu Ile Ala Glu His Gln Gly
    50                  55                  60

Glu Leu Trp Val Ala Ser Pro Leu Lys Lys Gln Arg Phe Arg Leu Ser
65                  70                  75                  80

Asp Gly Leu Cys Met Glu Asp Glu Gln Phe Ser Val Lys His Tyr Glu
                85                  90                  95

Ala Arg Val Lys Asp Gly Val Val Gln Leu Arg Gly
            100                 105

<210>  13
<211>  7320
<212>  DNA
<213>  Escherichia coli

<400>  13
gatcacgcaa aagtagaatt gtgcaaataa acggcagggt aatttttgaa ggtcagcaac      60

aaaagttgat taattctttg aggaacatgc agttatgcat gctgttgaaa aagaggaaga     120

tactgactaa ctctaaagtg gtattttaca tgcacttaca attgattaaa gacaacattc     180

acagtgtggt tatttgttac acataggggc gagcaatgtc atgacagtgt aggtgcggtt     240

actgtcgtga aaaacaataa aaaccgccat tgcaacaatg gcgcaattcg gatgaagccc     300

ctatgacaag gataaaaata aacgcacgcc gtatcttcag cttattgatt ccttttttct     360

ttttcacttc tgttcacgct gaacaaacgg ctgctcccgc aaaacctgta actgtggaag     420

cgaagaatga aacctttgcc ccgcagcatc ccgatcaata tctctcctgg aaagccacct     480

cggaacagtc agagcgtgtt gacgccctgg cggaagatcc acggctggtg atcctgtggg     540

cggggtatcc cttctcgcgc gattacaaca agccgcgtgg acatgctttt gctgtgaccg     600

atgtgcgtga aaccctgcgt accggtgcgc cgaaaaacgc tgaagatggt ccgctaccga     660

tggcatgctg gagttgtaaa agcccggatg tggcgcgtct gatccagaaa gacggcgaag     720

atggctactt ccacggtaaa tgggcgcgcg gcggtccgga aatcgtcaac aacttaggtt     780

gtgccgattg ccataacacc gcctctccag agttcgccaa aggcaaaccg gagttaaccc     840

tttcccgtcc gtatgcggct cgcgcgatgg aagccattgg taaacctttt gagaaagccg     900

gacgtttcga ccagcaatcg atggtttgcg gtcagtgcca tgtggagtat tacttcgacg 960
gcaaaaacaa agcggttaaa ttcccgtggg atgacggcat gaaagtcgaa aatatggagc 1020
agtattacga caaaattgcc ttctctgact ggactaactc cctgtcgaaa acgccaatgc 1080
tgaaagcgca gcacccggaa tatgaaacct ggacagcggg cattcacggt aaaaacaacg 1140
tgacctgtat cgactgccat atgccaaaag tgcagaacgc cgaaggcaaa ctctacaccg 1200
accataaaat tggtaatccg tttgataact tcgcccagac ttgtgcgaac tgccataccc 1260
aggacaaagc tgccttgcaa aaagtggtcg cggaacgtaa gcagtcgatt aacgacctga 1320
aaatcaaggt tgaagatcaa ctggttcacg ctcacttcga agcgaaagca gcgctggatg 1380
caggcgcgac ggaagctgaa atgaagccaa ttcaggacga tatccgtcat gcccagtggc 1440
gctgggatct ggcgatcgct tcccacggca ttcatatgca cgcaccggaa gaaggtttac 1500
ggatgctcgg tacggcgatg gataaagcgg cggatgcacg caccaaactg gcgcgcctgc 1560
tggcgaccaa aggcatcacc catgaaatcc agatcccgga tatctcaacc aaagagaaag 1620
cccagcaggc cattggcctg aacatggaac aaatcaaggc cgagaagcag gacttcatca 1680
aaacggtgat cccgcagtgg gaagaacagg cacgtaaaaa cggtctgtta agccaataac 1740
cccgttccgc ctcgcaaggg gcggaaaaca caatggagtg aatatgagcg tattacgttc 1800
gttattaact gccggggtgc tggcgtcggg cctgttgtgg agcctgaacg gaattaccgc 1860
tacccctgcg gcgcaggcat ctgatgatcg ctacgaagtt acccagcagc gtaacccgga 1920
tgccgcctgt ctggactgtc ataaaccaga taccgaaggt atgcatggaa aacatgcttc 1980
cgttatcaac ccgaataaca aactgccggt cacctgcacc aactgtcacg gccagccatc 2040
accgcaacac cgcgaagggg tgaaagatgt gatgcgcttt aacgagccga tgtacaaggt 2100
tggagagcag aacagcgtct gtatgtcctg tcatctgccg gaacagttgc aaaaagcgtt 2160
ctggccgcac gatgtccacg taaccaaagt ggcgtgcgcc agctgccatt ccctgcatcc 2220
acagcaagat acgatgcaga cgttaagtga caaaggacgg attaagattt gcgtcgattg 2280
ccacagcgat cagcgcacca atccgaactt taacccggcg tctgttccgt tgcttaagga 2340
gcagccatga cctggtctcg tcgccagttt ctcaccggcg tcggcgtgct ggcagccgtc 2400
agcggcaccg cagggcgcgt ggtggcgaaa acgttgaata tcaatggggt gcgttacggc 2460
atggtgcatg acgagtcgtt atgcatcggc tgtaccgcct gtatggatgc ttgtcgggaa 2520
gtgaacaagg tgccggaagg cgtctcgcgc ctgacgatta ttcgtagcga gccgcagggc 2580
gaatttcctg atgtgaaata tcgcttcttc cgtaagtctt gccagcactg cgatcatgcg 2640
ccgtgcgttg acgtctgccc gaccggtgcg tcttttcgcg atgctgccag tgggattgtc 2700
gatgttaatc cggatctctg cgtcggttgt cagtactgca tcgccgcctg tccgtaccgc 2760
gtgcgcttta tccatccggt cacgaagacg gcggacaaat gcgatttctg ccgtaagacc 2820
aatttgcagg ccggtaagtt gcccgcgtgc gttgaagctt gcccgaccaa ggcgctgacg 2880
tttggcaatc tggacgatcc caacagtgag atttcgcaac tgctgcgcca gaagcccact 2940
taccgctaca agctggcgct gggaaccaaa ccgaagctgt accgcgtacc gtttaaatac 3000
ggggaggtga gccaatgacg cagacttccg catttcattt tgaatcgctg gtgtgggact 3060
ggccgattgc catctacctg tttttgattg gtatttctgc tggtctggtg acgctggccg 3120

tgctgttacg tcgcttctac ccgcaggcgg gcggtgcaga cagtacgttg ctgcgcacca 3180
cgctgattgt cgggccgggc gcggtgatcc tcggtctgtt gatcctcgtc ttccacctga 3240
caagaccgtg gaccttctgg aagctgatgt tccactacag ttttacctcg gtgatgtcga 3300
tgggggtgat gctgtttcag ctctacatgg tggtgctggt gctgtggctg gcgaaaatct 3360
ttgaacatga tttgcttgcc ctgcaacaac gctggttgcc gaagctgggg atcgtgcaaa 3420
aggttctgag cctcgtgacg cccgttcatc gcggactgga aacattgatg ctggtgctgg 3480
cggtgttgtt gggggcttat accggctttc tgctgtcggc gctgaaatcg tatccgttcc 3540
tcaataaccc gatcctgccg gtgctgttcc tcttctccgg catctcgtcc ggtgcggcgg 3600
tggcgctgat cgccatgcgg atacgccaac gcagtaaccc gcattccacg gaagcgcagt 3660
ttgtacaccg tatggaaatc cctgtggtat ggggtgaaat cttcctgctg gtggcgtttt 3720
ttgtcggtct ggcgctgggc gatgacggta aagttcgtgc gctggtggcg gcattaggtg 3780
gcggtttctg gacgtggtgg ttctggcttg gtgtcgccgg gctggggctg attgtgccaa 3840
tgctgctcaa accgtgggtc aatcgcagtt ccggcattcc tgccgtgctg gcggcgtgtg 3900
gggccagtct ggtcggcgtg ttgatgctgc gctttttcat tctctacgcc ggacagttga 3960
cggtggcgta agccagaaaa gaggtggttt ctggacgtat tccttcctga agtcggtttt 4020
ctggcgttgt tgttaagtct cggggtcaac gtgttgaccc cgttgacggc cttcgcggga 4080
gtgcggttgc gctggcctgc catgatgcga ctcacttgca tcggcattct ggcgcagttc 4140
gccgtcctgc tgctcgcctt tgcggtactg acgtattgtt ttctcatcag cgatttctcg 4200
gtcatttatg tcgcgcaaca tagctatagc ctgctgtcgt gggaactcaa gctggcagcg 4260
gtgtggggcg gccatgaagg ttcgctgctg ctttgggtgc tgctgctttc cgcctggagc 4320
gcgctgtttg cctggcatta tcggcagcaa accgatccgc tatttccgct gacgttagcc 4380
gttttatctc tcatgctcgc cgcactgcta ctgtttgtgg tgctgtggtc cgatcccttc 4440
gtgcggatat ttccaccagc aatcgaaggc cgcgatctca atccgatgct gcaacatccc 4500
ggtcttatct ttcatccacc gctgctttac cttggctatg gcggtttgat ggtagcggcg 4560
agcgtggcgc tggcgagtct actgcgcggc gagtttgatg gtgcctgcgc ccgaatttcg 4620
tggcgctggg cgttacctgg ctggagtgca ttaacggcgg ggatcatcct cggttcctgg 4680
tgggcctact gcgaactcgg cctggggcgg ctggtggttc tgggatccgt ggaaaacgcc 4740
tctttattac cctggctttc tgccactgcg ctgctgcaca gtttgtccct gacacgccag 4800
cgggggattt tttgccactg gtcgctgttg ctggcgatag ttactctgat gctgtcgctg 4860
ctgggcacct taattgtccg ttctggcatt ctggtttcgg ttcatgcgtt cgcgctggat 4920
aacgtccgcg ccgtgccgtt gttcagcctg tttgcactga ttagccttgc gtctctggct 4980
ctgtatggct ggcgagcgcg ggacggtggc ccggcggtgc atttttcggg gttatcgcgg 5040
gaaatgttaa tcctcgctac gctgttgctg ttttgcgcag tgctactgat cgtgctggtg 5100
ggaacgcttt atccgatgat ttacggcctg ctgggctggg gacgcctctc cgttggcgcg 5160
ccgtatttta accgcgcgac gttaccgttt ggtctgttga tgctggtggt gattgtgctg 5220
gcgacgtttg tctctggcaa acgcgtgcag cttccggcgc tggtagctca tgcgggcgtg 5280
ctgttatttg ccgctggggt cgtggtctcc agcgtcagcc gtcaggagat cagcctgaat 5340
ttacagccgg gtcagcaggt gacgctggca ggatacacct tccgttttga gtgcctcgat 5400

ctacaagcca aagcgaatta caccagcgaa aaagcgatag tggcactgtt tgaccatcag 5460
caacgcattg gtgaactgac gccggagcgg gctttttatg aagcacgccg tcaacaaatg 5520
atggaaccgt caattcgctg gaacggcatc catgactggt atgcggtcat gggggagaaa 5580
actgggccgg atcgttacgc ttttcgtttg tatgtacaaa gcggtgtgcg ctggatctgg 5640
gggggaggat tgttgatgat tgcgggcgca ttgttaagcg gatggcgggg gaagaagcgc 5700
gatgaataaa gggcttctca cgctactgct gttatttacc tgttttgcgc acgctcaggt 5760
cgtagacacc tggcaattcg ccaatccgca acaacagcaa caggcgttaa atattgccag 5820
ccagttacgt tgtccgcagt gccagaatca aaacttactg gaatccaacg cgccggtggc 5880
tgtcagtatg cgccatcagg tttacagcat ggtggcggag gggaagaacg aagtcgaaat 5940
cattggctgg atgaccgaac gctacggaga ttttgttcgc tataacccac cgttaacggg 6000
tcagacgctg gtgttatggg cgctgccagt ggtgttgtta ctgctgatgg cactgatcct 6060
ctggcgagtg agggcgaagc gatgaaacag cccaaaatac cggtgaaaat gctgacaaca 6120
ctcacgatat tgatggtatt tctctgtgtc ggcagttatc tgttaagccc aaaatggcag 6180
gcggtacgtg cggagtatca gcgtcagcgc gatccgctac atcagtttgc cagccagcaa 6240
accccggaag cgcagcttca ggcattgcag gataaaatcc gcgctaatcc acaaaacagc 6300
gaacagtggg cgttactggg cgagtactat ctgtggcaaa acgattacag caattcgttg 6360
ctggcgtacc gtcaggcgtt gcaactacgt ggtgagaacg ctgaactgta tgcggcgctg 6420
gcgacggtgc tttattacca ggccagccag catatgaccg cccagactcg cgcaatgatc 6480
gacaaagccc tcgcgctgga cagtaatgaa atcaccgccc tgatgctgct ggcttccgat 6540
gcgtttatgc aggcgaacta cgcgcaagcc atcgaactat ggcaaaaagt gatggatctc 6600
aactcaccgc gagttaaccg aacacagctg gttgagtcga ttaatatggc gaaattgttg 6660
cagcggagat tggattaatc atctggtgat gaagattatg tcattgagct gcataaaaaa 6720
taatcgaatg aacatatgcc aaaaataatc actaatcagt attattgcag attaaacaaa 6780
taaaaatctt tccataacaa atggttattc attaatcctg ccataaactc gcgttattat 6840
gcattaatgc agcgaaaagc tctgttgtta aagggttgcg caacataccg cgcaaatgat 6900
actgatcata agcgttaaaa aaatctacaa accaacgcaa cacaattcat gccctggcag 6960
tatgtcacgt tctcgcgttt ctgaacgggg aacggcgctc cattgaggaa gtcattcata 7020
tgaaaaatat aaaattcagc ctggcctggc agattctgtt tgctatggtg ctgggcattc 7080
tcctgggaag ctacctgcac taccatagcg acagccgcga ctggctggtc gtcaatttgc 7140
tctctccggc gggtgatatc ttcatccatc tgattaaaat gattgttgtg ccgattgtga 7200
tctccacgct ggtggtgggt atcgcgggtg ttggtgatgc caaacagctc gggcgtattg 7260
gcgcgaaaac cattatctac ttcgaggtga tcaccaccgt cgccatcatt ttggggatcc 7320

<210> 14
<211> 450
<212> PRT
<213> Escherichia coli

<400> 14

Met Thr Arg Ile Lys Ile Asn Ala Arg Arg Ile Phe Ser Leu Leu Ile
1               5                   10                  15

Pro Phe Phe Phe Phe Thr Ser Val His Ala Glu Gln Thr Ala Ala Pro
            20                  25                  30

Ala Lys Pro Val Thr Val Glu Ala Lys Asn Glu Thr Phe Ala Pro Gln
        35                  40                  45

His Pro Asp Gln Tyr Leu Ser Trp Lys Ala Thr Ser Glu Gln Ser Glu
    50                  55                  60

Arg Val Asp Ala Leu Ala Glu Asp Pro Arg Leu Val Ile Leu Trp Ala
65                  70                  75                  80

Gly Tyr Pro Phe Ser Arg Asp Tyr Asn Lys Pro Arg Gly His Ala Phe
                85                  90                  95

Ala Val Thr Asp Val Arg Glu Thr Leu Arg Thr Gly Ala Pro Lys Asn
            100                 105                 110

Ala Glu Asp Gly Pro Leu Pro Met Ala Cys Trp Ser Cys Lys Ser Pro
        115                 120                 125

Asp Val Ala Arg Leu Ile Gln Lys Asp Gly Glu Asp Gly Tyr Phe His
    130                 135                 140

Gly Lys Trp Ala Arg Gly Gly Pro Glu Ile Val Asn Asn Leu Gly Cys
145                 150                 155                 160

Ala Asp Cys His Asn Thr Ala Ser Pro Glu Phe Ala Lys Gly Lys Pro
                165                 170                 175

Glu Leu Thr Leu Ser Arg Pro Tyr Ala Ala Arg Ala Met Glu Ala Ile
            180                 185                 190

Gly Lys Pro Phe Glu Lys Ala Gly Arg Phe Asp Gln Gln Ser Met Val
        195                 200                 205

Cys Gly Gln Cys His Val Glu Tyr Tyr Phe Asp Gly Lys Asn Lys Ala
    210                 215                 220

Val Lys Phe Pro Trp Asp Asp Gly Met Lys Val Glu Asn Met Glu Gln
225                 230                 235                 240

Tyr Tyr Asp Lys Ile Ala Phe Ser Asp Trp Thr Asn Ser Leu Ser Lys
                245                 250                 255

Thr Pro Met Leu Lys Ala Gln His Pro Glu Tyr Glu Thr Trp Thr Ala
            260                 265                 270

Gly Ile His Gly Lys Asn Asn Val Thr Cys Ile Asp Cys His Met Pro
        275                 280                 285

Lys Val Gln Asn Ala Glu Gly Lys Leu Tyr Thr Asp His Lys Ile Gly
    290                 295                 300

Asn Pro Phe Asp Asn Phe Ala Gln Thr Cys Ala Asn Cys His Thr Gln

305                 310                 315                 320

Asp Lys Ala Ala Leu Gln Lys Val Val Ala Glu Arg Lys Gln Ser Ile
                325                 330                 335

Asn Asp Leu Lys Ile Lys Val Glu Asp Gln Leu Val His Ala His Phe
            340                 345                 350

Glu Ala Lys Ala Ala Leu Asp Ala Gly Ala Thr Glu Ala Glu Met Lys
        355                 360                 365

Pro Ile Gln Asp Asp Ile Arg His Ala Gln Trp Arg Trp Asp Leu Ala
    370                 375                 380

Ile Ala Ser His Gly Ile His Met His Ala Pro Glu Glu Gly Leu Arg
385                 390                 395                 400

Met Leu Gly Thr Ala Met Asp Lys Ala Ala Asp Ala Arg Thr Lys Leu
                405                 410                 415

Ala Arg Leu Leu Ala Thr Lys Gly Ile Thr His Glu Ile Gln Ile Pro
            420                 425                 430

Asp Ile Ser Thr Lys Glu Lys Ala Gln Gln Ala Ile Gly Leu Asn Met
        435                 440                 445

Glu Gln
    450

<210>  15
<211>  160
<212>  PRT
<213>  Escherichia coli

<400>  15

Met Ser Val Leu Arg Ser Leu Leu Thr Ala Gly Val Leu Ala Ser Gly
1               5                   10                  15

Leu Leu Trp Ser Leu Asn Gly Ile Thr Ala Thr Pro Ala Ala Gln Ala
            20                  25                  30

Ser Asp Asp Arg Tyr Glu Val Thr Gln Gln Arg Asn Pro Asp Ala Ala
        35                  40                  45

Cys Leu Asp Cys His Lys Pro Asp Thr Glu Gly Met His Gly Lys His
    50                  55                  60

Ala Ser Val Ile Asn Pro Asn Asn Lys Leu Pro Val Thr Cys Thr Asn
65                  70                  75                  80

Cys His Gly Gln Pro Ser Pro Gln His Arg Glu Gly Val Lys Asp Val
                85                  90                  95

Met Arg Phe Asn Glu Pro Met Tyr Lys Val Gly Glu Gln Asn Ser Val
            100                 105                 110

Cys Met Ser Cys His Leu Pro Glu Gln Leu Gln Lys Ala Phe Trp Pro

        115                 120                 125

His Asp Val His Val Thr Lys Val Ala Cys Ala Ser Cys His Ser Leu
    130                 135                 140

His Pro Gln Gln Asp Thr Met Gln Thr Leu Ser Asp Lys Gly Arg Ile
145                 150                 155                 160

<210> 16
<211> 218
<212> PRT
<213> Escherichia coli

<400> 16

Met Thr Trp Ser Arg Arg Gln Phe Leu Thr Gly Val Gly Val Leu Ala
1               5                   10                  15

Ala Val Ser Gly Thr Ala Gly Arg Val Val Ala Lys Thr Leu Asn Ile
            20                  25                  30

Asn Gly Val Arg Tyr Gly Met Val His Asp Glu Ser Leu Cys Ile Gly
        35                  40                  45

Cys Thr Ala Cys Met Asp Ala Cys Arg Glu Val Asn Lys Val Pro Glu
    50                  55                  60

Gly Val Ser Arg Leu Thr Ile Ile Arg Ser Glu Pro Gln Gly Glu Phe
65                  70                  75                  80

Pro Asp Val Lys Tyr Arg Phe Phe Arg Lys Ser Cys Gln His Cys Asp
                85                  90                  95

His Ala Pro Cys Val Asp Val Cys Pro Thr Gly Ala Ser Phe Arg Asp
            100                 105                 110

Ala Ala Ser Gly Ile Val Asp Val Asn Pro Asp Leu Cys Val Gly Cys
        115                 120                 125

Gln Tyr Cys Ile Ala Ala Cys Pro Tyr Arg Val Arg Phe Ile His Pro
    130                 135                 140

Val Thr Lys Thr Ala Asp Lys Cys Asp Phe Cys Arg Lys Thr Asn Leu
145                 150                 155                 160

Gln Ala Gly Lys Leu Pro Ala Cys Val Glu Ala Cys Pro Thr Lys Ala
                165                 170                 175

Leu Thr Phe Gly Asn Leu Asp Asp Pro Asn Ser Glu Ile Ser Gln Leu
            180                 185                 190

Leu Arg Gln Lys Pro Thr Tyr Arg Tyr Lys Leu Ala Leu Gly Thr Lys
        195                 200                 205

Pro Lys Leu Tyr Arg Val Pro Phe Lys Tyr
    210                 215

<210> 17

<211> 318
<212> PRT
<213> Escherichia coli

<400> 17

Met Thr Gln Thr Ser Ala Phe His Phe Glu Ser Leu Val Trp Asp Trp
1               5                   10                  15

Pro Ile Ala Ile Tyr Leu Phe Leu Ile Gly Ile Ser Ala Gly Leu Val
            20                  25                  30

Thr Leu Ala Val Leu Leu Arg Arg Phe Tyr Pro Gln Ala Gly Gly Ala
        35                  40                  45

Asp Ser Thr Leu Leu Arg Thr Thr Leu Ile Val Gly Pro Gly Ala Val
    50                  55                  60

Ile Leu Gly Leu Leu Ile Leu Val Phe His Leu Thr Arg Pro Trp Thr
65                  70                  75                  80

Phe Trp Lys Leu Met Phe His Tyr Ser Phe Thr Ser Val Met Ser Met
                85                  90                  95

Gly Val Met Leu Phe Gln Leu Tyr Met Val Val Leu Val Leu Trp Leu
            100                 105                 110

Ala Lys Ile Phe Glu His Asp Leu Leu Ala Leu Gln Gln Arg Trp Leu
        115                 120                 125

Pro Lys Leu Gly Ile Val Gln Lys Val Leu Ser Leu Val Thr Pro Val
    130                 135                 140

His Arg Gly Leu Glu Thr Leu Met Leu Val Leu Ala Val Leu Leu Gly
145                 150                 155                 160

Ala Tyr Thr Gly Phe Leu Leu Ser Ala Leu Lys Ser Tyr Pro Phe Leu
                165                 170                 175

Asn Asn Pro Ile Leu Pro Val Leu Phe Leu Phe Ser Gly Ile Ser Ser
            180                 185                 190

Gly Ala Ala Val Ala Leu Ile Ala Met Arg Ile Arg Gln Arg Ser Asn
        195                 200                 205

Pro His Ser Thr Glu Ala Gln Phe Val His Arg Met Glu Ile Pro Val
    210                 215                 220

Val Trp Gly Glu Ile Phe Leu Leu Val Ala Phe Phe Val Gly Leu Ala
225                 230                 235                 240

Leu Gly Asp Asp Gly Lys Val Arg Ala Leu Val Ala Ala Leu Gly Gly
                245                 250                 255

Gly Phe Trp Thr Trp Trp Phe Trp Leu Gly Val Ala Gly Leu Gly Leu
            260                 265                 270

Ile Val Pro Met Leu Leu Lys Pro Trp Val Asn Arg Ser Ser Gly Ile

275                    280                    285

Pro Ala Val Leu Ala Ala Cys Gly Ala Ser Leu Val Gly Val Leu Met
    290                    295                    300

Leu Arg Phe Phe Ile Leu Tyr Ala Gly Gln Leu Thr Val Ala
305                    310                    315

<210>  18
<211>  552
<212>  PRT
<213>  Escherichia coli

<400>  18

Met Leu Thr Pro Leu Thr Ala Phe Ala Gly Val Arg Leu Arg Trp Pro
1               5                   10                  15

Ala Met Met Arg Leu Thr Cys Ile Gly Ile Leu Ala Gln Phe Ala Val
            20                  25                  30

Leu Leu Leu Ala Phe Ala Val Leu Thr Tyr Cys Phe Leu Ile Ser Asp
        35                  40                  45

Phe Ser Val Ile Tyr Val Ala Gln His Ser Tyr Ser Leu Leu Ser Trp
    50                  55                  60

Glu Leu Lys Leu Ala Ala Val Trp Gly Gly His Glu Gly Ser Leu Leu
65                  70                  75                  80

Leu Trp Val Leu Leu Leu Ser Ala Trp Ser Ala Leu Phe Ala Trp His
                85                  90                  95

Tyr Arg Gln Gln Thr Asp Pro Leu Phe Pro Leu Thr Leu Ala Val Leu
            100                 105                 110

Ser Leu Met Leu Ala Ala Leu Leu Leu Phe Val Val Leu Trp Ser Asp
        115                 120                 125

Pro Phe Val Arg Ile Phe Pro Pro Ala Ile Glu Gly Arg Asp Leu Asn
    130                 135                 140

Pro Met Leu Gln His Pro Gly Leu Ile Phe His Pro Pro Leu Leu Tyr
145                 150                 155                 160

Leu Gly Tyr Gly Gly Leu Met Val Ala Ala Ser Val Ala Leu Ala Ser
                165                 170                 175

Leu Leu Arg Gly Glu Phe Asp Gly Ala Cys Ala Arg Ile Ser Trp Arg
            180                 185                 190

Trp Ala Leu Pro Gly Trp Ser Ala Leu Thr Ala Gly Ile Ile Leu Gly
        195                 200                 205

Ser Trp Trp Ala Tyr Cys Glu Leu Gly Leu Gly Arg Leu Val Val Leu
    210                 215                 220

Gly Ser Val Glu Asn Ala Ser Leu Leu Pro Trp Leu Ser Ala Thr Ala

225                 230                 235                 240

Leu Leu His Ser Leu Ser Leu Thr Arg Gln Arg Gly Ile Phe Cys His
                245                 250                 255

Trp Ser Leu Leu Leu Ala Ile Val Thr Leu Met Leu Ser Leu Leu Gly
            260                 265                 270

Thr Leu Ile Val Arg Ser Gly Ile Leu Val Ser Val His Ala Phe Ala
        275                 280                 285

Leu Asp Asn Val Arg Ala Val Pro Leu Phe Ser Leu Phe Ala Leu Ile
    290                 295                 300

Ser Leu Ala Ser Leu Ala Leu Tyr Gly Trp Arg Ala Arg Asp Gly Gly
305                 310                 315                 320

Pro Ala Val His Phe Ser Gly Leu Ser Arg Glu Met Leu Ile Leu Ala
                325                 330                 335

Thr Leu Leu Leu Phe Cys Ala Val Leu Leu Ile Val Leu Val Gly Thr
            340                 345                 350

Leu Tyr Pro Met Ile Tyr Gly Leu Leu Gly Trp Gly Arg Leu Ser Val
        355                 360                 365

Gly Ala Pro Tyr Phe Asn Arg Ala Thr Leu Pro Phe Gly Leu Leu Met
    370                 375                 380

Leu Val Val Ile Val Leu Ala Thr Phe Val Ser Gly Lys Arg Val Gln
385                 390                 395                 400

Leu Pro Ala Leu Val Ala His Ala Gly Val Leu Leu Phe Ala Ala Gly
                405                 410                 415

Val Val Val Ser Ser Val Ser Arg Gln Glu Ile Ser Leu Asn Leu Gln
            420                 425                 430

Pro Gly Gln Gln Val Thr Leu Ala Gly Tyr Thr Phe Arg Phe Glu Cys
        435                 440                 445

Leu Asp Leu Gln Ala Lys Ala Asn Tyr Thr Ser Glu Lys Ala Ile Val
    450                 455                 460

Ala Leu Phe Asp His Gln Gln Arg Ile Gly Glu Leu Thr Pro Glu Arg
465                 470                 475                 480

Ala Phe Tyr Glu Ala Arg Arg Gln Gln Met Met Glu Pro Ser Ile Arg
                485                 490                 495

Trp Asn Gly Ile His Asp Trp Tyr Ala Val Met Gly Glu Lys Thr Gly
            500                 505                 510

Pro Asp Arg Tyr Ala Phe Arg Leu Tyr Val Gln Ser Gly Val Arg Trp
        515                 520                 525

Ile Trp Gly Gly Gly Leu Leu Met Ile Ala Gly Ala Leu Leu Ser Gly
530                 535                 540

Trp Arg Gly Lys Lys Arg Asp Glu
545                 550

<210>  19
<211>  127
<212>  PRT
<213>  Escherichia coli

<400>  19

Met Asn Lys Gly Leu Leu Thr Leu Leu Leu Leu Phe Thr Cys Phe Ala
1               5                   10                  15

His Ala Gln Val Val Asp Thr Trp Gln Phe Ala Asn Pro Gln Gln Gln
            20                  25                  30

Gln Gln Ala Leu Asn Ile Ala Ser Gln Leu Arg Cys Pro Gln Cys Gln
        35                  40                  45

Asn Gln Asn Leu Leu Glu Ser Asn Ala Pro Val Ala Val Ser Met Arg
    50                  55                  60

His Gln Val Tyr Ser Met Val Ala Glu Gly Lys Asn Glu Val Glu Ile
65                  70                  75                  80

Ile Gly Trp Met Thr Glu Arg Tyr Gly Asp Phe Val Arg Tyr Asn Pro
                85                  90                  95

Pro Leu Thr Gly Gln Thr Leu Val Leu Trp Ala Leu Pro Val Val Leu
            100                 105                 110

Leu Leu Leu Met Ala Leu Ile Leu Trp Arg Val Arg Ala Lys Arg
        115                 120                 125

<210>  20
<211>  198
<212>  PRT
<213>  Escherichia coli

<400>  20

Met Lys Gln Pro Lys Ile Pro Val Lys Met Leu Thr Thr Leu Thr Ile
1               5                   10                  15

Leu Met Val Phe Leu Cys Val Gly Ser Tyr Leu Leu Ser Pro Lys Trp
            20                  25                  30

Gln Ala Val Arg Ala Glu Tyr Gln Arg Gln Arg Asp Pro Leu His Gln
        35                  40                  45

Phe Ala Ser Gln Gln Thr Pro Glu Ala Gln Leu Gln Ala Leu Gln Asp
    50                  55                  60

Lys Ile Arg Ala Asn Pro Gln Asn Ser Glu Gln Trp Ala Leu Leu Gly
65                  70                  75                  80

Glu Tyr Tyr Leu Trp Gln Asn Asp Tyr Ser Asn Ser Leu Leu Ala Tyr

85                  90                  95

Arg Gln Ala Leu Gln Leu Arg Gly Glu Asn Ala Glu Leu Tyr Ala Ala
            100                 105                 110

Leu Ala Thr Val Leu Tyr Tyr Gln Ala Ser Gln His Met Thr Ala Gln
        115                 120                 125

Thr Arg Ala Met Ile Asp Lys Ala Leu Ala Leu Asp Ser Asn Glu Ile
    130                 135                 140

Thr Ala Leu Met Leu Leu Ala Ser Asp Ala Phe Met Gln Ala Asn Tyr
145                 150                 155                 160

Ala Gln Ala Ile Glu Leu Trp Gln Lys Val Met Asp Leu Asn Ser Pro
                165                 170                 175

Arg Val Asn Arg Thr Gln Leu Val Glu Ser Ile Asn Met Ala Lys Leu
            180                 185                 190

Leu Gln Arg Arg Leu Asp
        195

<210>  21
<211>  1686
<212>  DNA
<213>  Escherichia coli

<220>
<221>  CDS
<222>  (1).. (1683)
<223>

<400>  21
atg aca aca acc acc gga agt gcc cgg cca gca cgt gcc gcc agg aag      48
Met Thr Thr Thr Thr Gly Ser Ala Arg Pro Ala Arg Ala Ala Arg Lys
1               5                   10                  15

cct aag ccc gaa ggc caa tgg aaa atc gac ggc acc gag ccg ctt aac      96
Pro Lys Pro Glu Gly Gln Trp Lys Ile Asp Gly Thr Glu Pro Leu Asn
            20                  25                  30

cat gcc gag gaa att aag caa gaa gaa ccc gct ttt gct gtc aag cag      144
His Ala Glu Glu Ile Lys Gln Glu Glu Pro Ala Phe Ala Val Lys Gln
        35                  40                  45

cgg gtc att gat att tac tcc aag cag ggt ttt tct tcc att gca ccg      192
Arg Val Ile Asp Ile Tyr Ser Lys Gln Gly Phe Ser Ser Ile Ala Pro
    50                  55                  60

gat gac att gcc cca cgc ttt aag tgg ttg ggc att tac acc cag cgt      240
Asp Asp Ile Ala Pro Arg Phe Lys Trp Leu Gly Ile Tyr Thr Gln Arg
65                  70                  75                  80

aag cag gat ctg ggc ggt gaa ctg acc ggt cag ctt cct gat gat gag      288
Lys Gln Asp Leu Gly Gly Glu Leu Thr Gly Gln Leu Pro Asp Asp Glu
                85                  90                  95

ctg cag gat gag tac ttc atg atg cgt gtg cgt ttt gat ggc gga ctg      336
Leu Gln Asp Glu Tyr Phe Met Met Arg Val Arg Phe Asp Gly Gly Leu
            100                 105                 110

gct tcc cct gag cgc ctg cgt gcc gtg ggt gaa att tct agg gat tat      384
Ala Ser Pro Glu Arg Leu Arg Ala Val Gly Glu Ile Ser Arg Asp Tyr
        115                 120                 125

gct cgt tcc acc gcg gac ttc acc gac cgc cag aac att cag ctg cac      432

Ala Arg Ser Thr Ala Asp Phe Thr Asp Arg Gln Asn Ile Gln Leu His
    130                 135                 140

tgg att cgt att gaa gat gtg cct gcg atc tgg gag aag cta gaa acc 480
Trp Ile Arg Ile Glu Asp Val Pro Ala Ile Trp Glu Lys Leu Glu Thr
145                 150                 155                 160

gtc gga ctg tcc acc atg ctt ggt tgc ggt gac gtt cca cgt gtt atc 528
Val Gly Leu Ser Thr Met Leu Gly Cys Gly Asp Val Pro Arg Val Ile
                165                 170                 175

ttg ggc tcc cca gtt tct ggc gta gct gct gaa gag ctg atc gat gcc 576
Leu Gly Ser Pro Val Ser Gly Val Ala Ala Glu Glu Leu Ile Asp Ala
            180                 185                 190

acc ccg gct atc gat gcg att cgt gag cgc tac cta gac aag gaa gag 624
Thr Pro Ala Ile Asp Ala Ile Arg Glu Arg Tyr Leu Asp Lys Glu Glu
        195                 200                 205

ttc cac aac ctt cct cgt aag ttt aag act gct atc act ggc aac cag 672
Phe His Asn Leu Pro Arg Lys Phe Lys Thr Ala Ile Thr Gly Asn Gln
    210                 215                 220

cgc cag gat gtt acc cac gaa atc cag gac gtt tcc ttc gtt cct tcg 720
Arg Gln Asp Val Thr His Glu Ile Gln Asp Val Ser Phe Val Pro Ser
225                 230                 235                 240

att cac cca gaa ttc ggc cca gga ttt gag tgc ttt gtg ggc ggt ggc 768
Ile His Pro Glu Phe Gly Pro Gly Phe Glu Cys Phe Val Gly Gly Gly
                245                 250                 255

ctg tcc acc aac cca atg ctt gct cag cca ctt ggt tct tgg att cca 816
Leu Ser Thr Asn Pro Met Leu Ala Gln Pro Leu Gly Ser Trp Ile Pro
            260                 265                 270

ctt gat gag gtt cca gaa gtg tgg gct ggc gtc gcc gga att ttc cgc 864
Leu Asp Glu Val Pro Glu Val Trp Ala Gly Val Ala Gly Ile Phe Arg
        275                 280                 285

gac tac ggc ttc cga cgc ctg cgt aac cgt gct cgc ctc aag ttc ttg 912
Asp Tyr Gly Phe Arg Arg Leu Arg Asn Arg Ala Arg Leu Lys Phe Leu
    290                 295                 300

gtg gca cag tgg ggt att gag aag ttc cgt gaa gtt ctt gag acc gaa 960
Val Ala Gln Trp Gly Ile Glu Lys Phe Arg Glu Val Leu Glu Thr Glu
305                 310                 315                 320

tac ctc gag cgc aag ctg atc gat ggc cca gtt gtt acc acc aac cct 1008
Tyr Leu Glu Arg Lys Leu Ile Asp Gly Pro Val Val Thr Thr Asn Pro
                325                 330                 335

ggc tac cgt gac cac att ggc att cac cca caa aag gac ggc aag ttc 1056
Gly Tyr Arg Asp His Ile Gly Ile His Pro Gln Lys Asp Gly Lys Phe
            340                 345                 350

tac ctc ggt gtg aag cca acc gtt gga cac acc acc ggt gag cag ctc 1104
Tyr Leu Gly Val Lys Pro Thr Val Gly His Thr Thr Gly Glu Gln Leu
        355                 360                 365

att gcc att gct gat gtt gca gaa aag cac ggc atc acc agg att cgt 1152
Ile Ala Ile Ala Asp Val Ala Glu Lys His Gly Ile Thr Arg Ile Arg
    370                 375                 380

acc acg gcg gaa aag gaa ctg ctc ttc ctc gat att gag aga aag aac 1200
Thr Thr Ala Glu Lys Glu Leu Leu Phe Leu Asp Ile Glu Arg Lys Asn
385                 390                 395                 400

ctt act acc gtt gca cgc gac ctg gat gaa atc gga ctg tac tct tca 1248
Leu Thr Thr Val Ala Arg Asp Leu Asp Glu Ile Gly Leu Tyr Ser Ser
                405                 410                 415

cct tcc gag ttc cgc cgc ggc atc att tcc tgc acc ggc ttg gag ttc 1296
Pro Ser Glu Phe Arg Arg Gly Ile Ile Ser Cys Thr Gly Leu Glu Phe
            420                 425                 430

tgc aag ctt gcg cac gca acc acc aag tca cga gca att gag ctt gtc     1344
Cys Lys Leu Ala His Ala Thr Thr Lys Ser Arg Ala Ile Glu Leu Val
        435                 440                 445

gac gaa ctg gaa gag cgc ctc ggc gat ttg gat gtt ccc atc aag att     1392
Asp Glu Leu Glu Glu Arg Leu Gly Asp Leu Asp Val Pro Ile Lys Ile
    450                 455                 460

gca ctg aac ggt tgc cct aac tct tgt gca cgc acc cag gtt tcc gac     1440
Ala Leu Asn Gly Cys Pro Asn Ser Cys Ala Arg Thr Gln Val Ser Asp
465                 470                 475                 480

atc gga ttc aag gga cag acc gtc act gat gct gac ggc aac cgc gtt     1488
Ile Gly Phe Lys Gly Gln Thr Val Thr Asp Ala Asp Gly Asn Arg Val
                485                 490                 495

gaa ggt ttc cag gtt cac ctg ggc ggt tcc atg aac ttg gat cca aac     1536
Glu Gly Phe Gln Val His Leu Gly Gly Ser Met Asn Leu Asp Pro Asn
            500                 505                 510

ttc gga cgc aag ctc aag ggc cac aag gtt att gcc gat gaa gtg gga     1584
Phe Gly Arg Lys Leu Lys Gly His Lys Val Ile Ala Asp Glu Val Gly
        515                 520                 525

gag tac gtc act cgc gtt gtt acc cac ttc aag gaa cag cgc cac gag     1632
Glu Tyr Val Thr Arg Val Val Thr His Phe Lys Glu Gln Arg His Glu
    530                 535                 540

gac gag cac ttc cgc gat tgg gtc cag cgg gcc gct gag gaa gat ttg     1680
Asp Glu His Phe Arg Asp Trp Val Gln Arg Ala Ala Glu Glu Asp Leu
545                 550                 555                 560

gtg tga                                                             1686
Val

<210>  22
<211>  561
<212>  PRT
<213>  Escherichia coli

<400>  22

Met Thr Thr Thr Thr Gly Ser Ala Arg Pro Ala Arg Ala Ala Arg Lys
1               5                   10                  15

Pro Lys Pro Glu Gly Gln Trp Lys Ile Asp Gly Thr Glu Pro Leu Asn
            20                  25                  30

His Ala Glu Glu Ile Lys Gln Glu Glu Pro Ala Phe Ala Val Lys Gln
        35                  40                  45

Arg Val Ile Asp Ile Tyr Ser Lys Gln Gly Phe Ser Ser Ile Ala Pro
    50                  55                  60

Asp Asp Ile Ala Pro Arg Phe Lys Trp Leu Gly Ile Tyr Thr Gln Arg
65                  70                  75                  80

Lys Gln Asp Leu Gly Gly Glu Leu Thr Gly Gln Leu Pro Asp Asp Glu
                85                  90                  95

Leu Gln Asp Glu Tyr Phe Met Met Arg Val Arg Phe Asp Gly Gly Leu
            100                 105                 110

Ala Ser Pro Glu Arg Leu Arg Ala Val Gly Glu Ile Ser Arg Asp Tyr
        115                 120                 125

Ala Arg Ser Thr Ala Asp Phe Thr Asp Arg Gln Asn Ile Gln Leu His
    130                 135                 140

Trp Ile Arg Ile Glu Asp Val Pro Ala Ile Trp Glu Lys Leu Glu Thr
145                 150                 155                 160

Val Gly Leu Ser Thr Met Leu Gly Cys Gly Asp Val Pro Arg Val Ile
                165                 170                 175

Leu Gly Ser Pro Val Ser Gly Val Ala Ala Glu Glu Leu Ile Asp Ala
            180                 185                 190

Thr Pro Ala Ile Asp Ala Ile Arg Glu Arg Tyr Leu Asp Lys Glu Glu
        195                 200                 205

Phe His Asn Leu Pro Arg Lys Phe Lys Thr Ala Ile Thr Gly Asn Gln
    210                 215                 220

Arg Gln Asp Val Thr His Glu Ile Gln Asp Val Ser Phe Val Pro Ser
225                 230                 235                 240

Ile His Pro Glu Phe Gly Pro Gly Phe Glu Cys Phe Val Gly Gly Gly
                245                 250                 255

Leu Ser Thr Asn Pro Met Leu Ala Gln Pro Leu Gly Ser Trp Ile Pro
            260                 265                 270

Leu Asp Glu Val Pro Glu Val Trp Ala Gly Val Ala Gly Ile Phe Arg
        275                 280                 285

Asp Tyr Gly Phe Arg Arg Leu Arg Asn Arg Ala Arg Leu Lys Phe Leu
    290                 295                 300

Val Ala Gln Trp Gly Ile Glu Lys Phe Arg Glu Val Leu Glu Thr Glu
305                 310                 315                 320

Tyr Leu Glu Arg Lys Leu Ile Asp Gly Pro Val Val Thr Thr Asn Pro
                325                 330                 335

Gly Tyr Arg Asp His Ile Gly Ile His Pro Gln Lys Asp Gly Lys Phe
            340                 345                 350

Tyr Leu Gly Val Lys Pro Thr Val Gly His Thr Thr Gly Glu Gln Leu
        355                 360                 365

Ile Ala Ile Ala Asp Val Ala Glu Lys His Gly Ile Thr Arg Ile Arg
    370                 375                 380

Thr Thr Ala Glu Lys Glu Leu Leu Phe Leu Asp Ile Glu Arg Lys Asn
385                 390                 395                 400

Leu Thr Thr Val Ala Arg Asp Leu Asp Glu Ile Gly Leu Tyr Ser Ser
                405                 410                 415

Pro Ser Glu Phe Arg Arg Gly Ile Ile Ser Cys Thr Gly Leu Glu Phe
            420                 425                 430

Cys Lys Leu Ala His Ala Thr Thr Lys Ser Arg Ala Ile Glu Leu Val
        435                 440                 445

Asp Glu Leu Glu Glu Arg Leu Gly Asp Leu Asp Val Pro Ile Lys Ile
    450                 455                 460

Ala Leu Asn Gly Cys Pro Asn Ser Cys Ala Arg Thr Gln Val Ser Asp
465                 470                 475                 480

Ile Gly Phe Lys Gly Gln Thr Val Thr Asp Ala Asp Gly Asn Arg Val
                485                 490                 495

Glu Gly Phe Gln Val His Leu Gly Gly Ser Met Asn Leu Asp Pro Asn
            500                 505                 510

Phe Gly Arg Lys Leu Lys Gly His Lys Val Ile Ala Asp Glu Val Gly
        515                 520                 525

Glu Tyr Val Thr Arg Val Val Thr His Phe Lys Glu Gln Arg His Glu
    530                 535                 540

Asp Glu His Phe Arg Asp Trp Val Gln Arg Ala Ala Glu Glu Asp Leu
545                 550                 555                 560

Val